# EUROPEAN PATENT APPLICATION

(11) **EP 1 466 631 A1**
(43) Date of publication of application: **13.10.2004**
(21) Application number: 02783596.6
(22) Date of filing: 21.11.2002
(51) Int. Cl.: A61L 27/00, A61K 45/00, A61K 9/52, A61K 9/70, A61K 31/4015, A61K 47/04, A61K 47/30, A61K 47/32, A61K 47/34, A61K 47/36, A61K 47/42, A61K 47/46, A61P 19/00, C07D 209/34

(54) **PREPARATIONS APPROPRIATE FOR CARTILAGE TISSUE FORMATION**

(30) Priority: 21.11.2001 JP 2001356662; 17.12.2001 JP 2001383622
(71) Applicant: CHUGAI SEIYAKU KABUSHIKI KAISHA, Tokyo, 115-8543 (JP)
(72) Inventor: SHIMOBOJI, Tsuyoshi c/o Chugai Seiyaku K.K., Gotenba-shi, Shizuoka 412-8513 (JP); KITAMURA, Hidetomo c/o Chugai Seiyaku K.K., Gotenba-shi, Shizuoka 412-8513 (JP); HAYASHI, Yoshiki c/o Chugai Seiyaku K.K., Gotenba-shi, Shizuoka 412-8513 (JP)
(74) Representative: HOFFMANN EITLE
(86) International application number: PCT/JP2002/012207
(87) International publication number: WO 2003/043672

(57) **Abstract**

A formulation for cartilage tissue formation, comprising a drug having a chondrogenesis-promoting action, a biodegradable and/or biocorrosive polymer, and a porous matrix and/or a hydrogel, wherein the porus matrix and the hydrogel substantially do not inhibit cartilage repair.

## Description

### Technical Field

The present invention relates to a formulation for cartilage tissue formation.

### Background Art

It is known that articular cartilage does not regenerate when damaged (Biomaterials 21 2589-2598 (2000)). Numerous methods have been proposed in the last 40 years or so for treatment of cartilage diseases such as articular cartilage loss, osteoarthritis and the like, and more recently it has been attempted to achieve cartilage regeneration by autologous cell transplantation, although this requires multiple operations, is inconvenient, and places a tremendous burden on patients. When implementing artificial joints, on the other hand, biocompatibility and durability have been significant issues to consider. Treatment methods with drugs that can solve these problems have therefore long been desired.

Various biologically derived substances and low molecular substances are known which have effects of promoting chondrogenesis or inducing proliferation of chondrocytes. As examples of such substances there may be mentioned growth factors such as TGF-β (Transforming Growth Factor β), BMP-2 (J. Bone Joint Surg. 79-A(10):1452-1463, 1997), concanavalin A, a kind of lectin (J. Biol. Chem., 265:10125-10131, 1990), osteogenin (BMP-7), vitamin D derivatives (1α, 25-D₃) (Cancer Res., 49:5435-5442, 1989), vitamin A derivatives (retinoic acid) (Dev. Biol., 130:767-773, 1988), vanadates (J. Cell Biol., 104:311-319, 1987), benzamide (J. Embryol. Exp. Morphol., 85:163-175, 1985), benzyl β-D-xyloside (Biochem. J., 224:977-988, 1984), triiodothyronine (T₃) (Endocrinology, 111:462-468, 1982), prostaglandin derivatives (PGE₂, U44069) (Prostaglandin, 19:391-406, 1980), dbcAMP (J. Cell. Physil., 100:63-76, 1979), 8-Br-cAMP (J. Cell. Physil., 100:63-76, 1979), and TAK-778 (Biochem. Biophys. Res. Com., 261:131-138, 1999). Also, WO00/44722 discloses that indolin-2-one derivatives with specific structures have chondrogenesis-promoting action.

TGF-β is considered to be the most promising as a treatment agent among such biologically derived substances and low molecular substances, and TGF-β₁, an isoform of TGF-β, has been reported to promote chondrogenesis when intraarticularly administered (Lab. Invest. 71(2):279-290, 1994). In experimental arthritis model animals, TGF-β₁ also suppresses arthritis-induced loss of proteoglycans in articular cartilage. Specifically, upon intraarticular administration it is assimilated into the articular cartilage and suppresses destruction of articular cartilage, suggesting its potential usefulness as a therapeutic agent for articular diseases such as rheumatism (Lab. Invest. 78(2):133-142, 1998).

However, even in the case of TGF-β which is expected to be most useful as a treatment agent, this particular substance has been reported to evoke marked synovitis while it promotes chondrogenesis. Consequently, serious problems exist for its use as a treatment agent for the above-mentioned cartilage diseases (Lab. Invest. 71(2):279-290, 1994) and therefore it has not been used in practice as a treatment agent for such diseases.

Several formulating methods have been hitherto proposed in order to avoid such side effects of drugs which promote chondrogenesis. For example, methods of adding chondrogenesis-promoting drugs to drug reservoirs for controlling release time have been reported, including methods of using biodegradable polymers and methods involving intraarticular administration of lactic acid/glycolic acid copolymer (PLGA) microcapsules containing chondrogenesis-promoting substances (Biochemical and Biophysical Research Communication 261, 131-138 (1999)), but these methods have been associated with risks such as inflammation at the site of administration and inhibition of natural repair. Methods of adding the drugs to liposomes (Japanese Patent Application Laid-Open No. 2001-302496) have been reported as means for avoiding such tissue irritancy, but these methods have been associated with other problems including shortened drug release times.

On the other hand, research is also being conducted on the use of matrices as scaffolds for cartilage tissue repair (for example, J. Orthopaedic Research, 17, 205-213 (1999)), and the reports to date have related to collagen sponges (Biomaterials, 17, 155-162 (1996)), gelatin sponges (J. Biomedical Material Research, 52, 246-255 (2000)), fibrin (Plastic and Reconstructive Surgery, 5, 1580-1585 (1998)), agarose gel (Osteoarthritis and Cartilage, 6, 50-65 (1998)), PLGA-polyethylene oxide (PEG) sponges (J. Biomedical Material Research 55(2), 229-235 (2001)), hyaluronic acid (Japanese Patent Application Laid-Open No. 2000-239305), transesterified hyaluronic acid (J. Biomedical Material Research 50, 101-109 (2000)) and chitosan-polysaccharide gels (Biomaterials, 21, 2589-2598 (2000)).

### Disclosure of the Invention

Because of the aforementioned problems inherent in the prior art, however, no formulation has yet existed which comprises a chondrogenesis-promoting drug as the active ingredient and has actually been marketed as a pharmaceutical.

The present inventors have already reported a formulation for cartilage tissue formation which comprises a drug having a chondrocyte proliferating action, a biodegradable and/or biocorrosive polymer and a water-soluble and/or water-dispersible organic solvent, as a means of solving the problems described above (WO01/66142). The formulation exhibits a highly superior chondrocyte proliferating action, but the proliferating action has been unstable in some cases.

The present invention has been accomplished in light of this and the aforementioned other problems of the prior art, and its object is to provide a formulation for cartilage tissue formation which is highly biocompatible, stable and capable of producing a satisfactory cartilage repair effect, without requiring highly skilled techniques or extensive equipment for treatment.

As a result of much research carried out with the aim of achieving the object described above, the present inventors have completed this invention upon finding that a formulation comprising a drug having a chondrogenesis-promoting action, a biodegradable and/or biocorrosive polymer and a porous matrix and/or hydrogel serving as a scaffold for cartilage tissue repair can independently control the drug release property, the biodegradability and/or biocorrosive property and the scaffold-forming property for cartilage tissue repair, and that the aforementioned object is thereby achieved.

In other words, the formulation for cartilage tissue formation of the present invention is characterized by comprising a drug having a chondrogenesis-promoting action, a biodegradable and/or biocorrosive polymer, and a porous matrix and/or a hydrogel, wherein the porus matrix and the hydrogel substantially do not inhibit cartilage repair. The polymer is a polymer which controls release of the drug *in vivo*, while the porous matrix and hydrogel are scaffold-forming materials which serve as scaffolding for cartilage tissue formation *in vivo*.

In the formulation for cartilage tissue formation of the invention, the drug and the polymer form a loaded-structure with the drug supported by the polymer, and the porous matrix and/or hydrogel (scaffold-forming material) are preferably in contact with the loaded-structure.

The loaded-structure used may be in the form of a microsphere, a film or nonwoven fabric, where the microsphere is preferably obtained from an aqueous dispersion of the drug and the polymer in an organic solvent solution, by removing the organic solvent and water. The loaded structure is preferably a loaded-structure capable of sustained release of the drug.

As a preferred mode of the formulation for cartilage tissue formation, there may be mentioned a dispersion of the loaded-structure in the aforementioned scaffold-forming material (where the scaffold-forming material is preferably a hydrogel) or a formed layer of the scaffold-forming material (where the scaffold-forming material is preferably a hydrogel) with the loaded-structure (where the loaded-structure is preferably in the form of a film or nonwoven fabric) fixed on the layer.

According to the invention, the drug is preferably a compound represented by the general formula (I) or a salt thereof. 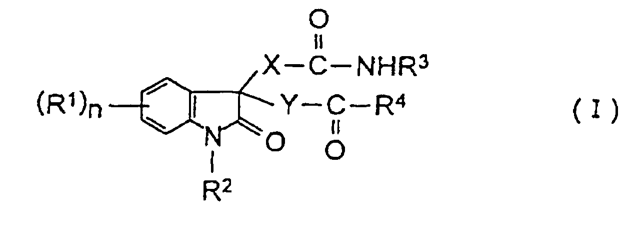 wherein
R¹ represents a halogen atom, a lower alkyl group, a lower alkoxy group, a hydroxyl group, a nitro group, a trifluoromethyl group, a lower alkylthio group, an acyl group, a carboxyl group, a mercapto group or an amino group with an optional substituent;
R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent or a heterocyclic group with an optional substituent;
R³ represents a lower alkyl group with an optional substituent, a cycloalkyl group with an optional substituent, an aryl group with an optional substituent or a heterocyclic group with an optional substituent;
R⁴ represents a hydrogen atom, a lower alkyl group with an optional substituent, an aryl group with an optional substituent, a heterocyclic group with an optional substituent, -OR⁵, -SR⁵ or -NR⁶R⁷ wherein R⁵, R⁶ and R⁷ may be the same or different and each represents a hydrogen atom, a lower alkyl group with an optional substituent, a cycloalkyl group with an optional substituent, an aryl group with an optional substituent, a heterocyclic group with an optional substituent, a lower alkoxy group or an amino group with an optional substituent, and R⁶ and R⁷ may together form a group represented by -(CH₂)ₘ- or -(CH₂)ₗNR⁸(CH₂)ₖ- wherein k, l and m each represent an integer of 1-8 and R⁸ represents a hydrogen atom or a lower alkyl group; and
X and Y may be the same or different and each represents -CH₂-, -NH- or -O-, and n represents an integer of 0-4.

A preferred compound represented by the general formula (I) is the compound represented by the formula (A) below.

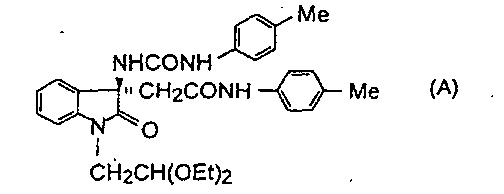

The polymer preferably has a weight-average molecular weight of 500 or greater, and as such polymer there may be mentioned one or more polymers selected from the group consisting of a polyhydroxyalkanoic acid, a lactone ring-opened polymer, a hydroxyalkanoic acid/lactone copolymer, a polyacetal, a polyketal, a polymethylvinyl ether, chitin, chitosan, a polyamide, a polyamino acid, a polyurethane, a polyester amide, a polycarboxylic acid, a polyanhydride, a polyalkylene, a polyphosphoric acid, a polyorthoester, and a copolymer comprising one or more of the foregoing in the molecule. Among these preferable is a polymer or a copolymer of a compound selected from the group consisting of glycolic acid, lactic acid, hydroxybutyric acid and hydroxyvaleric acid. The polymer is most preferably a lactic acid/glycolic acid copolymer.

According to the invention, at least a part of the polymer may be a reactive polymer having an unsaturated double bond. As such reactive polymers there may be mentioned a compound comprising a backbone and at least one (meth)acryloyl group bonded to the backbone, wherein the backbone comprises one or more polymers selected from the group consisting of a polyhydroxyalkanoic acid, a lactone ring-opened polymer, a hydroxyalkanoic acid/lactone copolymer, a polyacetal, a polyketal, a polymethylvinyl ether, chitin, chitosan, a polyamide, a polyamino acid, a polyurethane, a polyester amide, a polycarboxylic acid, a polyanhydride, a polyalkylene, a polyphosphoric acid, a polyorthoester, and a copolymer comprising one or more of the foregoing in the molecule.

A porous matrix according to the invention is preferably a porous matrix having a mean pore size of 10-500 µm, and is also preferably a porous matrix having an *in vivo* elimination rate of 1 day to 5 weeks.

The porous matrix may be a porous matrix comprising a biodegradable and/or biocorrosive compound, and the compound preferably has a weight-average molecular weight of 500 or greater.

As biodegradable and/or biocorrosive compounds there may be mentioned a hyaluronic acid derivative and/or collagen. Examples of the hyaluronic acid derivative include physically crosslinked hyaluronic acid or chemically crosslinked hyaluronic acid with low-crosslinked density, and examples of physically crosslinked hyaluronic acid include hydrogen bond-type physically crosslinked hyaluronic acid or ionic bond-type physically crosslinked hyaluronic acid.

As biodegradable and/or biocorrosive compounds there may also be mentioned one or more polymers selected from the group consisting of a polyhydroxyalkanoic acid, a lactone ring-opened polymer, a hydroxyalkanoic acid/lactone copolymer, a polyacetal, a polyketal, a polymethylvinyl ether, chitin, chitosan, a polyamide, a polyamino acid, a polyurethane, a polyester amide, a polycarboxylic acid, a polyanhydride, a polyalkylene, a polyphosphoric acid, a polyorthoester, and a copolymer comprising one or more of the foregoing in the molecule. Among there preferable is a polymer or a copolymer of a compound selected from the group consisting of glycolic acid, lactic acid, hydroxybutyric acid and hydroxyvaleric acid. Lactic acid/glycolic acid copolymer is particularly preferred as the biodegradable and/or biocorrosive compound.

At least a part of the biodegradable and/or biocorrosive compound may be a reactive polymer having an unsaturated double bond, and as such reactive polymers there may be mentioned a compound comprising a backbone and at least one (meth)acryloyl group bonded to the backbone, wherein the backbone comprises one or more polymers selected from the group consisting of a polyhydroxyalkanoic acid, a lactone ring-opened polymer, a hydroxyalkanoic acid/lactone copolymer, a polyacetal, a polyketal, a polymethylvinyl ether, chitin, chitosan, a polyamide, a polyamino acid, a polyurethane, a polyester amide, a polycarboxylic acid, a polyanhydride, a polyalkylene, a polyphosphoric acid, a polyorthoester, and a copolymer comprising one or more of the foregoing in the molecule.

The hydrogel according to the invention is preferably a hydrogel having an *in vivo* elimination rate of 1 day to 5 weeks. The hydrogel also preferably is a hydrogel capable of gelling *in situ,* and as such materials there may be mentioned the biodegradable and/or biocorrosive compounds referred to above.

The present invention also provides, in addition to the above, a method of forming cartilage tissue comprising introducing into a cartilage defect lesion a formulation for cartilage tissue formation having the loaded-structure dispersed in a scaffold-forming material (where the scaffold-forming material is preferably a hydrogel), and further provides a method of forming cartilage tissue comprising introducing into a cartilage defect lesion a formulation for cartilage tissue formation having a formed layer of the scaffold-forming material (where the scaffold-forming material is preferably a hydrogel) with the loaded-structure (where the loaded structure is preferably in the form of a film or nonwoven fabric) fixed on the layer, in such a manner that the layer is in contact with the surface of the cartilage defect lesion.

### Brief Description of the Drawings

Fig. 1 is a scanning electron micrograph of a porous matrix produced using a lactic acid/glycolic acid copolymer sponge (Example 2-2).
Fig. 2 is a scanning electron micrograph of a porous matrix produced using a collagen I sponge (Example 2-3).
Fig. 3 is a scanning electron micrograph of a porous matrix produced using a collagen II sponge (Example 2-4).
Fig. 4 is a scanning electron micrograph of a porous matrix produced using a chemically crosslinked HA sponge (Comparative Example 2-2).
Fig. 5 is a graph showing the results of a mesenchymal stem cell proliferation test using porous matrices and hydrogels of the examples and comparative examples.
Fig. 6 is a micrograph showing a Safranin O-Fast Green stained tissue sample of normal rabbit articular cartilage used for evaluation of a porous matrix.
Fig. 7 is a micrograph showing a Safranin O-Fast Green stained tissue sample of artificially damaged rabbit articular cartilage without implantation of either a porous matrix or hydrogel.
Fig..8 is a micrograph showing a Safranin O-Fast Green stained tissue sample of artificially damaged rabbit articular cartilage after 4 weeks of implanting the hydrogel of Example 2-1.
Fig. 9 is a micrograph showing a Safranin O-Fast Green stained tissue sample of artificially damaged rabbit articular cartilage after 4 weeks of implanting the porous matrix of Example 2-2.
Fig. 10 is a micrograph showing a Safranin O-Fast Green stained tissue sample of artificially damaged rabbit articular cartilage after 4 weeks of implanting the hydrogel of Comparative Example 2-1.
Fig. 11 is a micrograph showing a Safranin O-Fast Green stained tissue sample of artificially damaged rabbit articular cartilage after 4 weeks of implanting the porous matrix of Comparative Example 2-2.
Fig. 12 is a scanning electron micrograph of microspheres comprising Compound A and PLGA7510.
Fig. 13 is a graph showing time-dependent changes in drug release for microspheres comprising Compound A and PLGA7510.
Fig. 14 is a graph showing the drug release rate for microspheres comprising Compound A and PLGA7510.
Fig. 15 is a graph showing the drug release rate for a film comprising Compound A and Medisorb7525.
Fig. 16 is a graph showing time-dependent change in the cumulative release rate of Compound A from a physically crosslinked hyaluronic acid hydrogel encapsulating microspheres comprising Compound A and Medisorb5050.
Fig. 17 is a graph showing time-dependent change in the cumulative release rate of Compound A from a film comprising Compound A and Medisorb7525.
Fig. 18 is a micrograph showing a Safranin O-Fast Green stained tissue sample of normal rabbit articular cartilage used for evaluation of a formulation for cartilage tissue formation according to the invention.
Fig. 19 is a micrograph showing a Safranin O-Fast Green stained tissue sample of artificially damaged rabbit articular cartilage without implantation of a formulation for cartilage tissue formation according to the invention.
Fig. 20 is a micrograph showing a Safranin O-Fast Green stained tissue sample of artificially damaged rabbit articular cartilage, 3 months after implanting a formulation prepared by placing the physically crosslinked hyaluronic acid hydrogel of Example 4-3 so as to fill the damaged site and situating thereover a PLGA film containing no Compound A.
Fig. 21 is a micrograph showing a Safranin O-Fast Green stained tissue sample of artificially damaged rabbit articular cartilage, 3 months after implanting a formulation prepared by placing the physically crosslinked hyaluronic acid hydrogel of Example 4-3 so as to fill the damaged site and situating thereover a PLGA film containing 1 mg of Compound A of Example 4-1.
Fig. 22 is a micrograph showing a Safranin O-Fast Green stained tissue sample of artificially damaged rabbit articular cartilage, 3 months after implanting into the damaged site a physically crosslinked hyaluronic acid hydrogel formulation encapsulating PLGA microspheres containing no Compound A.
Fig. 23 is a micrograph showing a Safranin O-Fast Green stained tissue sample of artificially damaged rabbit articular cartilage, 3 months after implanting into the damaged site a physically crosslinked hyaluronic acid hydrogel formulation encapsulating the microspheres of Example 4-4 containing 1 mg of Compound A.

### Best Mode for Carrying Out the Invention

The present invention will now be explained in greater detail with reference to the accompanying drawings where necessary. In the following description, the "parts" and "%" values for the amount ratios are based on weight, unless otherwise specified.

As mentioned above, the formulation for cartilage tissue formation according to the invention comprises a drug having a chondrogenesis-promoting action, a biodegradable and/or biocorrosive polymer and a porous matrix which substantially does not inhibit cartilage tissue repair and/or a hydrogel which substantially does not inhibit cartilage repair.

### (Drug having chondrogenesis-promoting action)

The drug having a chondrogenesis-promoting action which is used for the invention may be a protein preparation, or a non-protein preparation such as a low molecular compound (.where "a low molecular compound" refers to a compound considered low molecular in the field, and includes, for example, an organic compound with molecular weight of about 1000 and lower). The chondrogenesis promoting action is believed to be brought by the ability to induce cartilage differentiation or to promote cartilage matrix production, and the chondrogenesis promoting action can be confirmed for example, by a method of measuring ³⁵S sulfate uptake by the method described in Anal. Biochem. 81:40-46, 1977 upon treatment of chondrocytes or cartilage precursor cells, or a method of assaying production of sulfated glycosaminoglycan based on the description in Connective Tissue Res. 9:247-248, 1982. Preferred drugs having chondrogenesis-promoting action are those which exhibit two-fold or greater action with respect to controls in the assay methods mentioned above.

As examples of drugs having chondrogenesis-promoting actions there may be mentioned growth factors such as TGF-β (Transforming Growth Factor β) and its isoforms (TGF-β₁, etc.), as well as BMP-2 (J. Bone Joint Surg. 79-A(10):1452-1463, 1997), concanavalin A, a kind of lectin (J. Biol. Chem., 265:10125-10131, 1990), osteogenin (BMP-7), vitamin D derivatives (1α, 25-D₃) (Cancer Res., 49:5435-5442, 1989), vitamin A derivatives (retinoic acid) (Dev. Biol., 130:767-773, 1988), vanadates (J. Cell Biol., 104:311-319, 1987), benzamide (J. Embryol. Exp. Morphol., 85:163-175, 1985), benzyl β-D-xyloside (Biochem. J., 224:977-988, 1984), triiodothyronine (T₃) (Endocrinology, 111:462-468, 1982), prostaglandin derivatives (PGE₂, U44069) (Prostaglandin, 19:391-406, 1980), dbcAMP (J. Cell. Physil., 100:63-76, 1979), 8-Br-cAMP (J. Cell. Physil., 100:63-76, 1979), TAK-778 (Biochem. Biophys. Res. Com., 261:131-138, 1999), compounds represented by general formula (I) above (indolin-2-one derivatives, WO00/44722), and the like.

Preferred as drugs having chondrogenesis-promoting action are compounds represented by the general formula (I) above and their salts. Compounds represented by the general formula (I) may be obtained, for example, according to the methods described in WO94/19322 (Japanese Patent Application Laid-Open No. HEI 7-48349), WO00/44722 and WO01/66142.

The compounds represented by the general formula (I) will now be explained, but first the terms "halogen", "lower alkyl", "lower alkenyl", "lower alkynyl", "lower alkoxy", "acyl", "aryl", "lower alkylene", "cycloalkyl" and "heterocyclic" used for the invention will be explained.

A "halogen atom" according to the invention is a fluorine atom, a chlorine atom, a bromine atom or an iodine atom.

A "lower alkyl group" according to the invention is a linear or branched alkyl group of 1 to 6 carbons, such as a methyl group, an ethyl group, a n-propyl group, an i-propyl group, a n-butyl group, a s-butyl group, a t-butyl group, a pentyl group, a hexyl group or the like.

A "lower alkenyl group" is a linear or branched alkenyl group of 2 to 6 carbons, such as a vinyl group, an allyl group, a butenyl group, a pentenyl group, a hexenyl group or the like.

A "lower alkynyl group" is a linear or branched alkynyl group of 2 to 6 carbons, such as an ethynyl group, a propynyl group, a butynyl group or the like.

A "lower alkoxy group" is a linear or branched alkoxy group of 1 to 6 carbons, such as a methoxy group, an ethoxy group, a n-propoxy group, an i-propoxy group, a n-butoxy group, a s-butoxy group, a t-butoxy group, a pentoxy group, a hexoxy group or the like.

An "acyl group" is a carbonyl group substituted with a hydrogen atom or with an alkyl group with an optional substituent, an aryl group with an optional substituent, an alkoxy group with an optional substituent, an amino group with an optional substituent or the like, for example, an alkylcarbonyl group such as an acetyl group, a propionyl group, a pivaloyl group, a cyclohexanecarbonyl group or the like, or an arylcarbonyl group such as a benzoyl group, a naphthoyl group, a toluoyl group or the like.

An "aryl group" is a monovalent group which is an aromatic hydrocarbon minus one hydrogen atom, such as a phenyl group, a tolyl group, a xylyl group, a biphenyl group, a naphthyl group, an anthryl group, a phenanthryl group or the like.

A "lower alkylene group" is a linear or branched alkylene group of 1 to 6 carbons, such as a methylene group, an ethylene group, a propylene group, a butylene group, a pentylene group, a hexylene group or the like.

A "cycloalkyl group" is a cyclic saturated hydrocarbon group of 3 to 8 carbons, such as a cyclopropyl group, a cyclobutyl group, a cyclopentyl group, a cyclohexyl group or a cycloheptyl group. And, substituted cycloalkyl groups include menthyl group, adamantyl group and the like.

A "heterocyclic group" is an aromatic heterocyclic group with at least one hetero atom, such as a pyridyl group, a furyl group, a thienyl group, an imidazolyl group, a pyrazinyl group, a pyrimidyl group or the like.

The aforementioned lower alkyl group, lower alkenyl group, alkynyl group, lower alkoxy group, acyl group, aryl group, cycloalkyl group and heterocyclic group may, if necessary, be substituted with one or more substituents. As examples of such substituents there may be a halogen atom, a lower alkyl group, a cycloalkyl group, an aryl group, a hydroxyl group, a lower alkoxy group which alkoxy group may be substituted with a halogen atom, an aryloxy group, a lower alkylthio group, a heterocyclic group, a formyl group which formyl group may be protected as an acetal, a lower alkylcarbonyl group, an arylcarbonyl group, a carboxyl group, a lower alkoxycarbonyl group, an amino group which amino group may have a lower alkyl group, etc., an imino group, a thioacetal group, a nitro group, a nitrile group, a trifluoromethyl group and the like.

The compounds serving as the active ingredients in the chondrogenesis promoters and cartilage repair agents of the invention (indolin-2-one derivatives represented by the general formula (I) above) will now be explained in greater detail.

R¹ represents a halogen atom, a lower alkyl group, a lower alkoxy group, a hydroxyl group, a nitro group, a trifluoromethyl group, a lower alkylthio group, an acyl group, a carboxyl group, a mercapto group or an amino group with an optional substituent, and among these, R¹ is preferably a halogen atom, a lower alkyl group, a lower alkoxy group or a nitro group.

The subscript "n" represents an integer of 0 to 4. It is preferably 0 or 1, and most preferably 0.

R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent or a heterocyclic group with an optional substituent.

R² is preferably a hydrogen atom, a lower alkyl group with an optional substituent, a lower alkenyl group with an optional substituent or an aryl group with an optional substituent, and from the viewpoint of a chondrocyte-proliferating action, it is even more preferably a lower alkyl group with an optional substituent which is optionally substituted with a halogen atom.

Among these, R² is yet more preferably a lower alkyl group substituted at the same carbon with two lower alkoxy groups which are optionally substituted with 1-5 halogen atoms or the group -O-Z-O- wherein Z represents a lower alkylene group optionally substituted with 1-10 halogen atoms, and still more preferably, a group represented by the general formula (II): 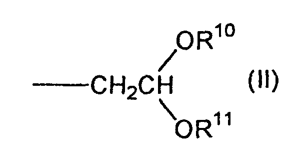 wherein R¹⁰ and R¹¹ may be the same or different, and each represents a lower alkyl group optionally substituted with 1-5 halogen atoms, preferably either or both being lower alkyl groups with 1-5 halogen atoms, or the general formula (III): 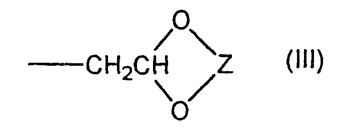 wherein Z represents a lower alkylene group optionally substituted with 1-10 halogen atoms.

R² is more preferably a 2,2-diethoxyethyl group, a 2,2-dimethoxyethyl group, a 2,2-diisopropoxyethyl group, a 2,2-bis(2-fluoroethoxy)ethyl group or a 2,2-bis(2-chloroethoxy)ethyl group, among which a 2,2-diethoxyethyl group and a 2,2-bis(2-fluoroethoxy)ethyl group are most preferred, and a 2,2-diethoxyethyl group is particularly preferred.

R³ represents a lower alkyl group with an optional substituent, a cycloalkyl group with an optional substituent, an aryl group with an optional substituent or a heterocyclic group with an optional substituent. R³ is preferably a lower alkyl group with an optional substituent, a cycloalkyl group with an optional substituent or an aryl group with an optional substituent, among which an aryl group with an optional substituent is particularly preferred. Preferred as the substituent is a lower alkyl group (preferably a methyl group and an ethyl group, and especially a. methyl group) and an amino group optionally having a lower alkyl group, and a 4-methylphenyl group is especially preferred for R³.

R⁴ represents a hydrogen atom, a lower alkyl group with an optional substituent, an aryl group with an optional substituent, a heterocyclic group with an optional substituent, -OR⁵, -SR⁵ or -NR⁶R⁷. Here, R⁵, R⁶ and R⁷ may be the same or different and each represents a hydrogen atom, a lower alkyl group with an optional substituent, a cycloalkyl group with an optional substituent, an aryl group with an optional substituent, a heterocyclic group with an optional substituent, a lower alkoxy group or an amino group with an optional substituent, and R⁶ and R⁷ may together form a group represented by -(CH₂)ₘ- or -(CH₂)ₗNR⁸(CH₂)ₖ- wherein k, l and m each represent an integer of 1-8 and R⁸ represents a hydrogen atom or a lower alkyl group.

R⁴ is preferably a lower alkyl group with an optional substituent, an aryl group with an optional substituent, a heterocyclic group with an optional substituent or a group represented by -OR⁵ or -NR⁶R⁷ wherein R⁵, R⁶ and R⁷ are as previously defined, and it is more preferably the group -NR⁶R⁷ wherein R⁶ and R⁷ may be the same or different and each represents a hydrogen atom or an aryl group with an optional substituent.

Among these, R⁴ is preferably a group represented by -NR⁶R⁷ wherein R⁶ and R⁷ may be the same or different and each represents a hydrogen atom or an aryl group which aryl group has a lower alkyl group or amino group which amino group optionally has a lower alkyl group, and it is most preferably a group represented by -NHR⁷ wherein R⁷ is a 4-methylphenyl group, a 4-ethylphenyl group or a 4-(N,N-dimethylamino)phenyl group.

X and Y may be the same or different and represent -CH₂-, -NH- or -O-, among which X is preferably -CH₂-, -NH- or -O- and Y is preferably -CH₂- or -NH-. From the viewpoint of a chondrocyte-proliferating action, X and Y may be the same or different and are preferably -CH₂- or -NH-, and most preferably, X is -NH- and Y is -CH₂-.

The indolin-2-one derivatives of the invention may be used in the form of pharmaceutically acceptable salts. As examples of such salts there may be mentioned inorganic salts such as hydrochloric acid salts, hydrobromic acid salts, hydroiodic acid salts, sulfuric acid salts and phosphoric acid salts; organic acid salts such as succinic acid salts, malonic acid salts, acetic acid salts, maleic acid salts, fumaric acid salts, citric acid salts, benzoic acid salts and salicylic acid salts; and metal salts such as sodium salts, potassium salts and magnesium salts.

The indolin-2-one derivatives of the invention may also be optically active forms. When in optically active forms, the absolute configuration at the 3 position is preferably the R configuration.

As examples of specific compounds of indolin-2-one derivatives according to the invention there may be mentioned the compounds mentioned in the examples of WO94/19322, as well as the compounds mentioned in the examples of Japanese Patent Application Laid-Open No. HEI 7-48349, namely, Compound Nos. 1-201 listed in the following tables (Tables 1-9). In Tables 1-9, R¹-R⁴, X, Y and n have the same definitions as for general formula (I) above.

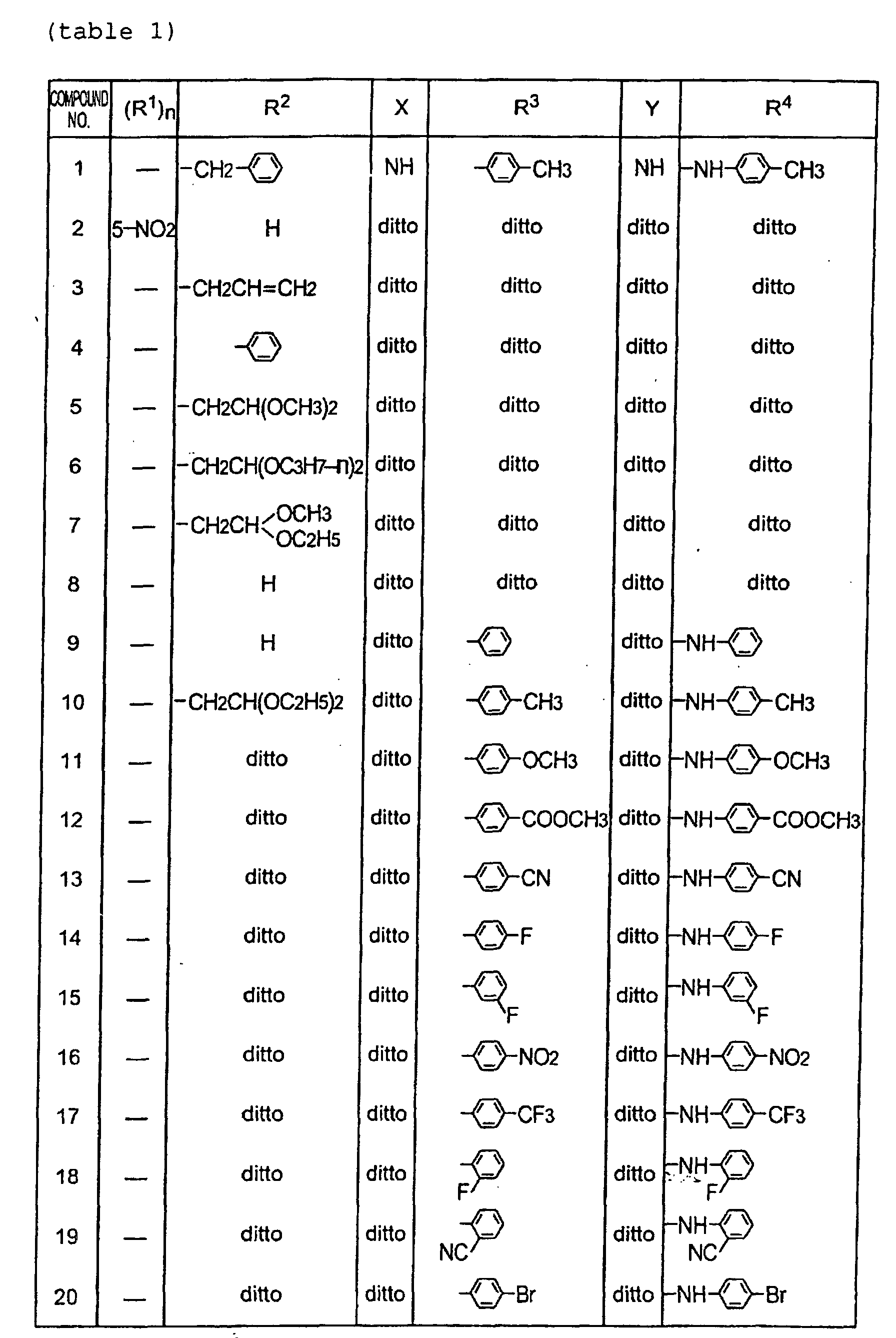

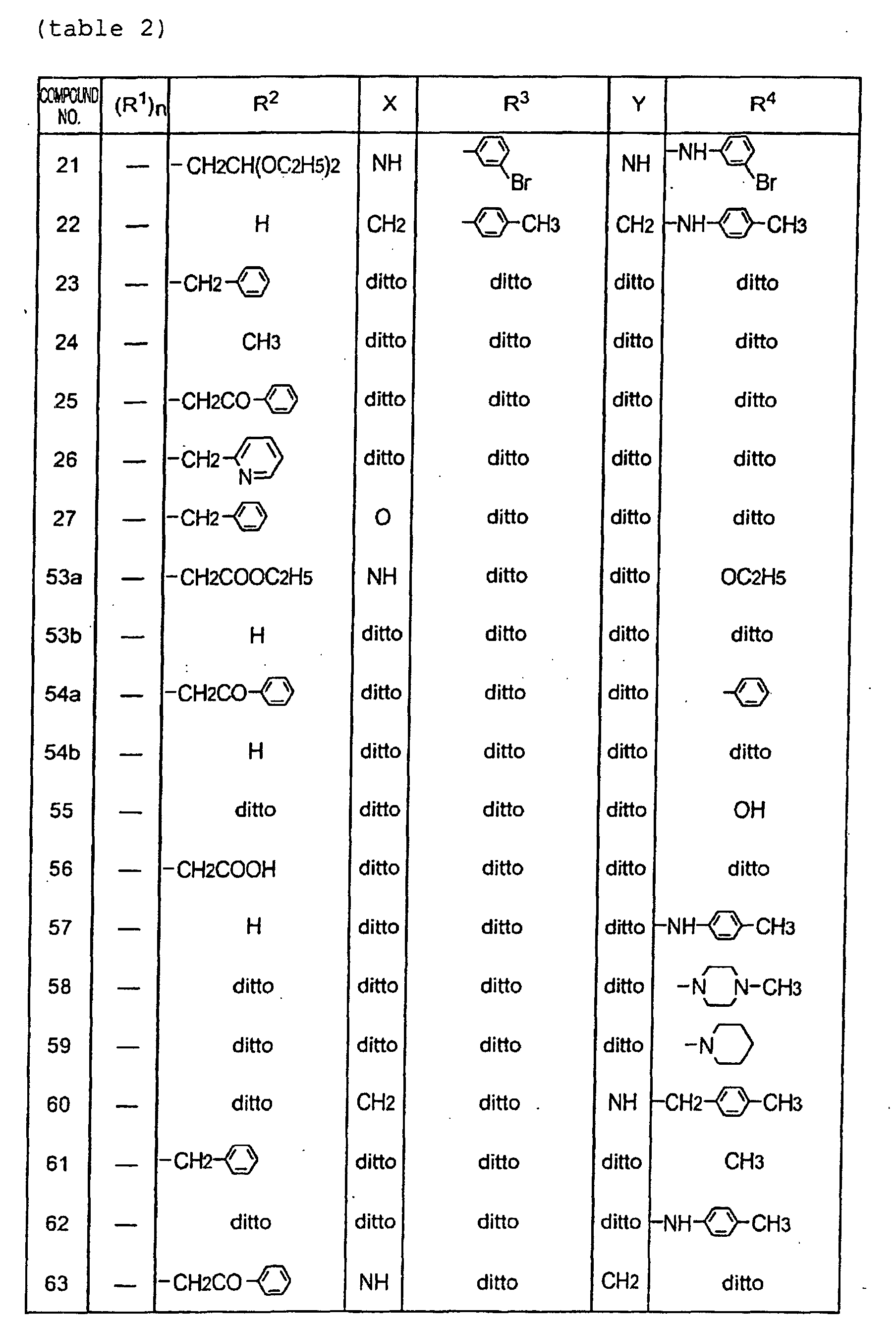

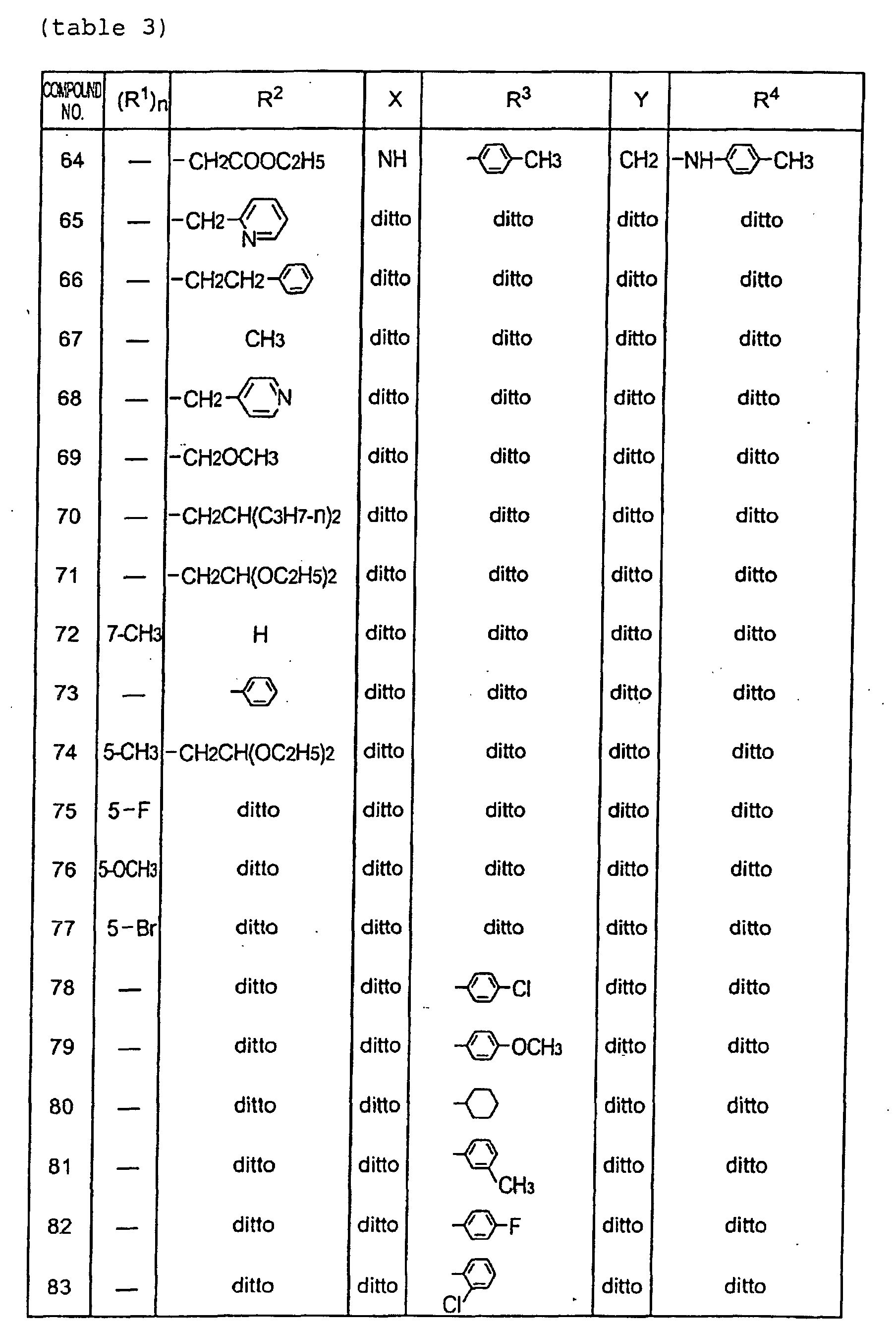

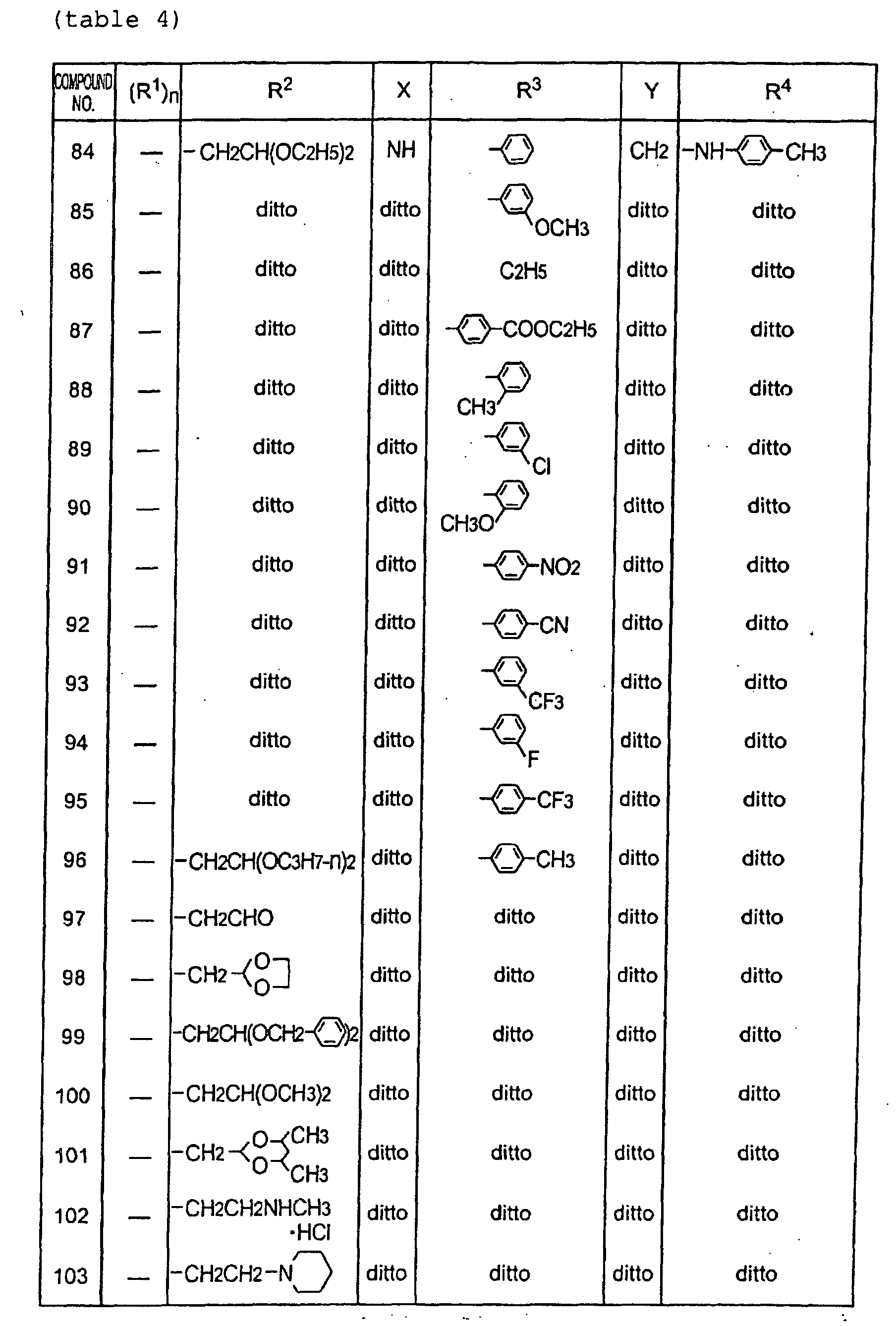

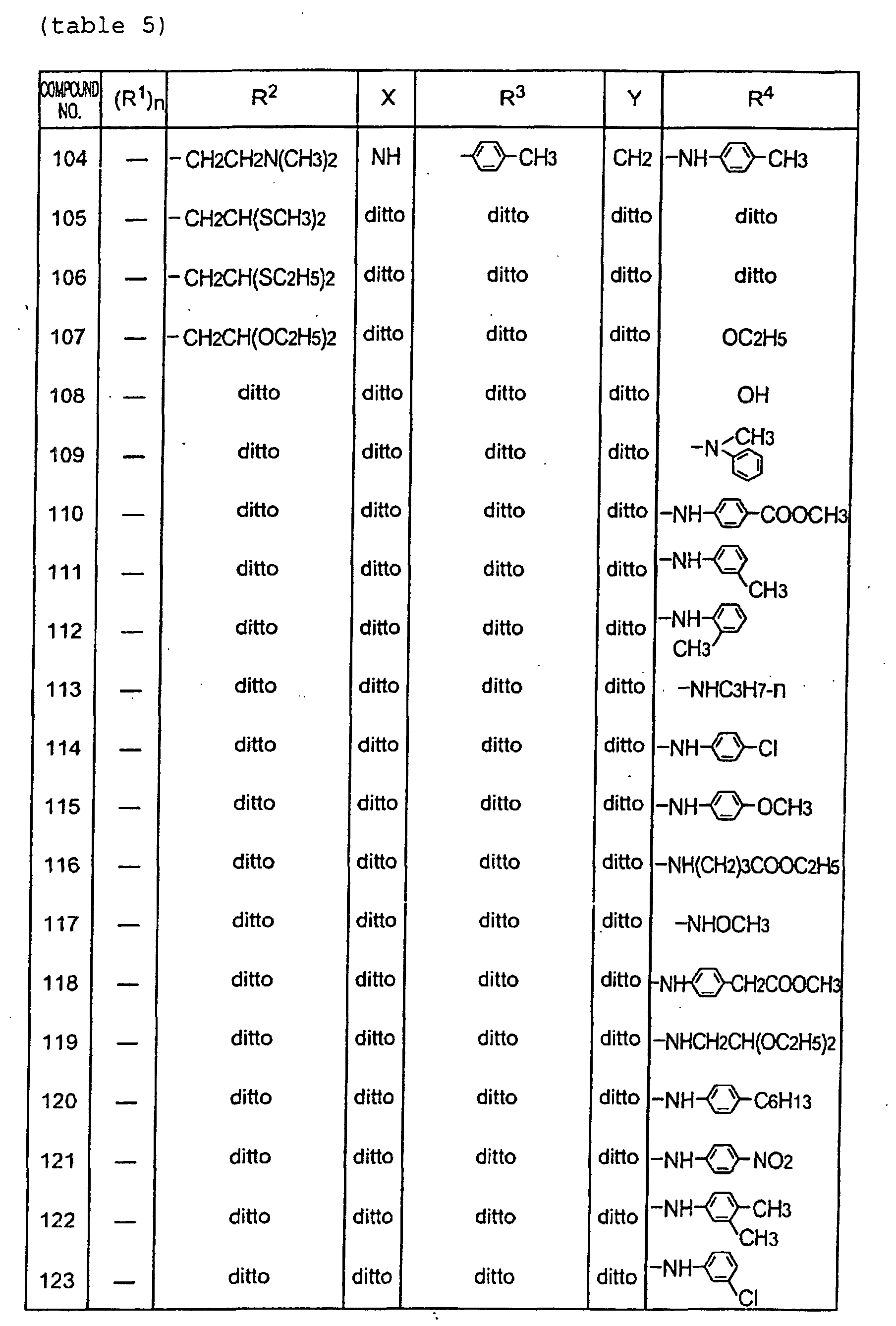

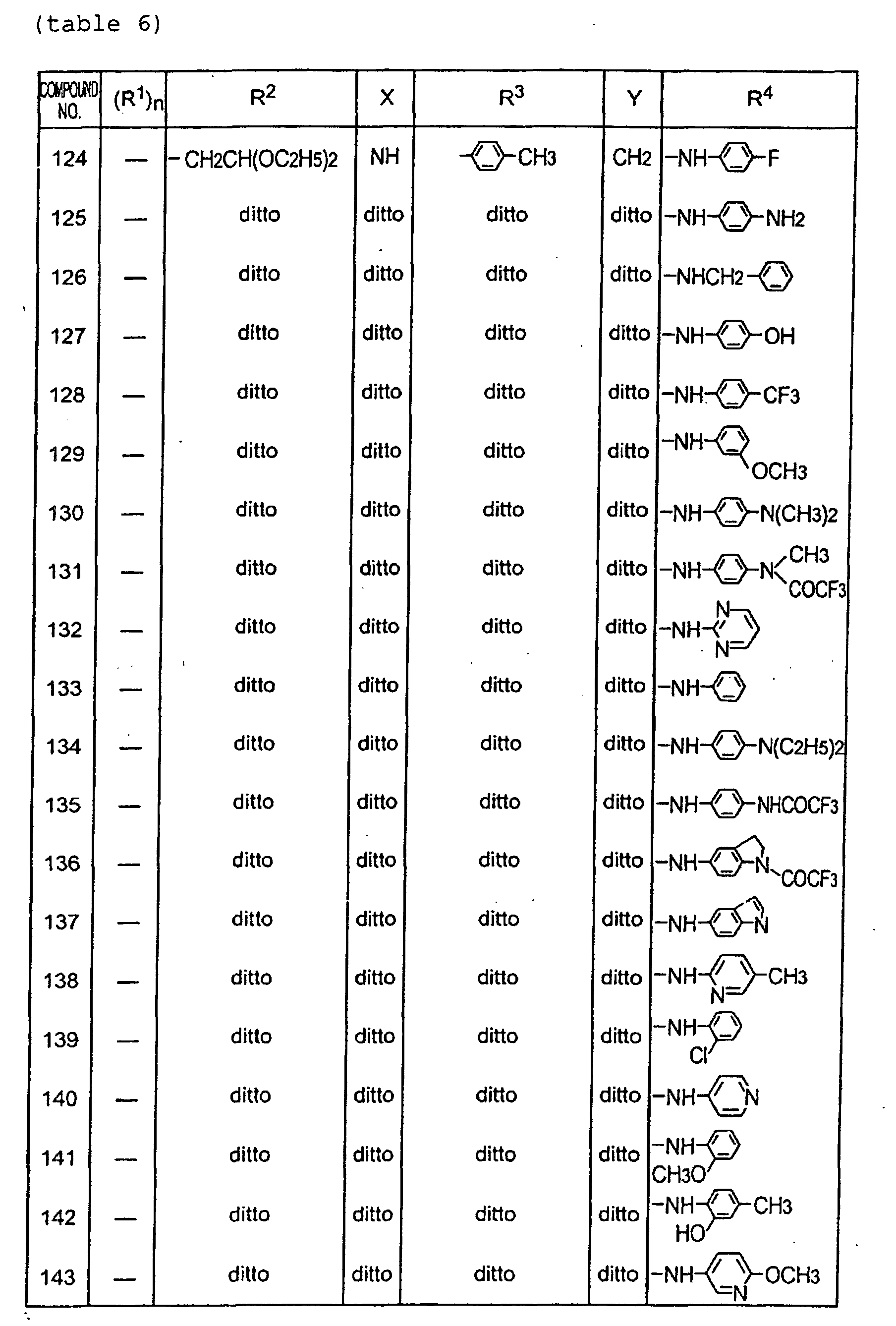

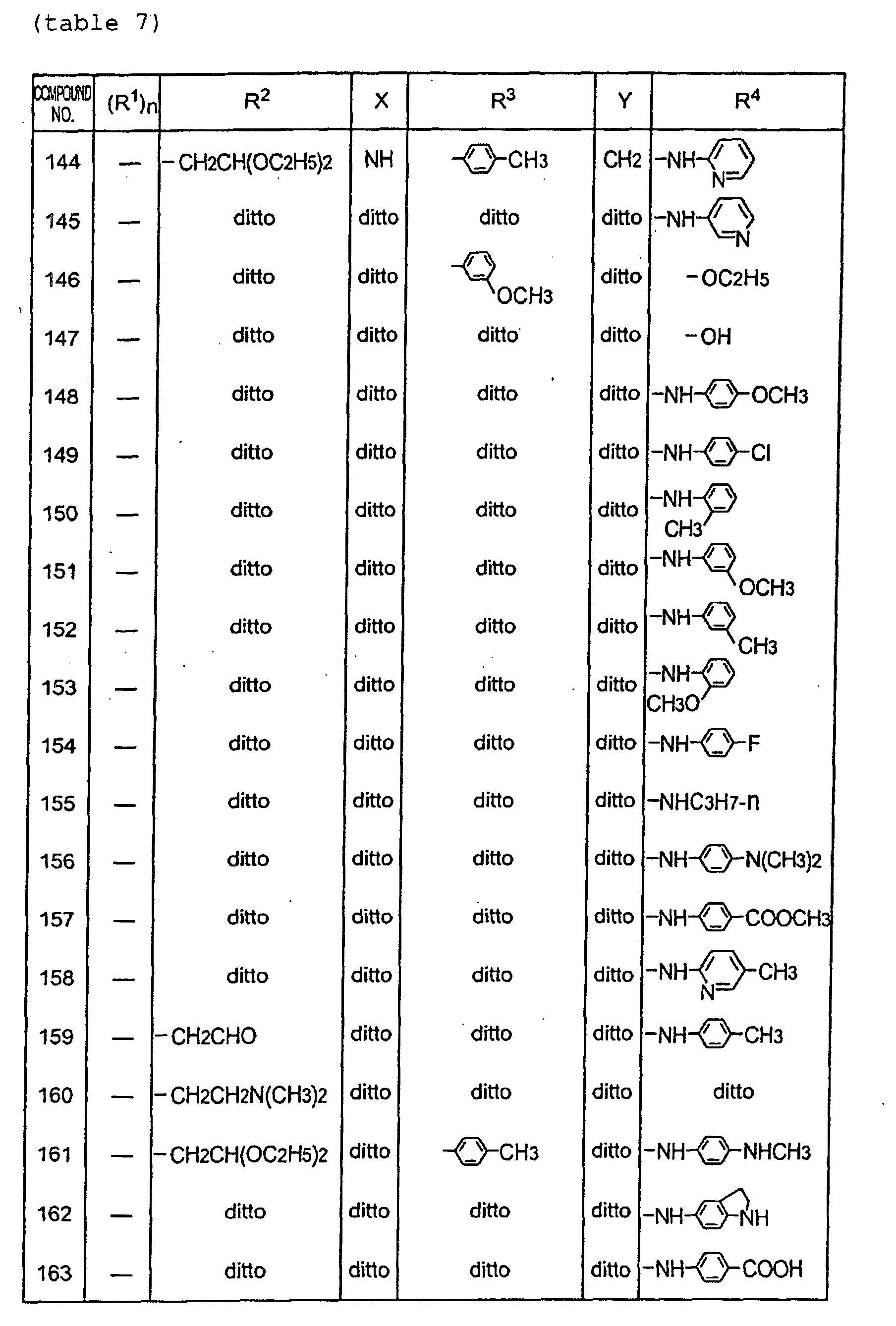

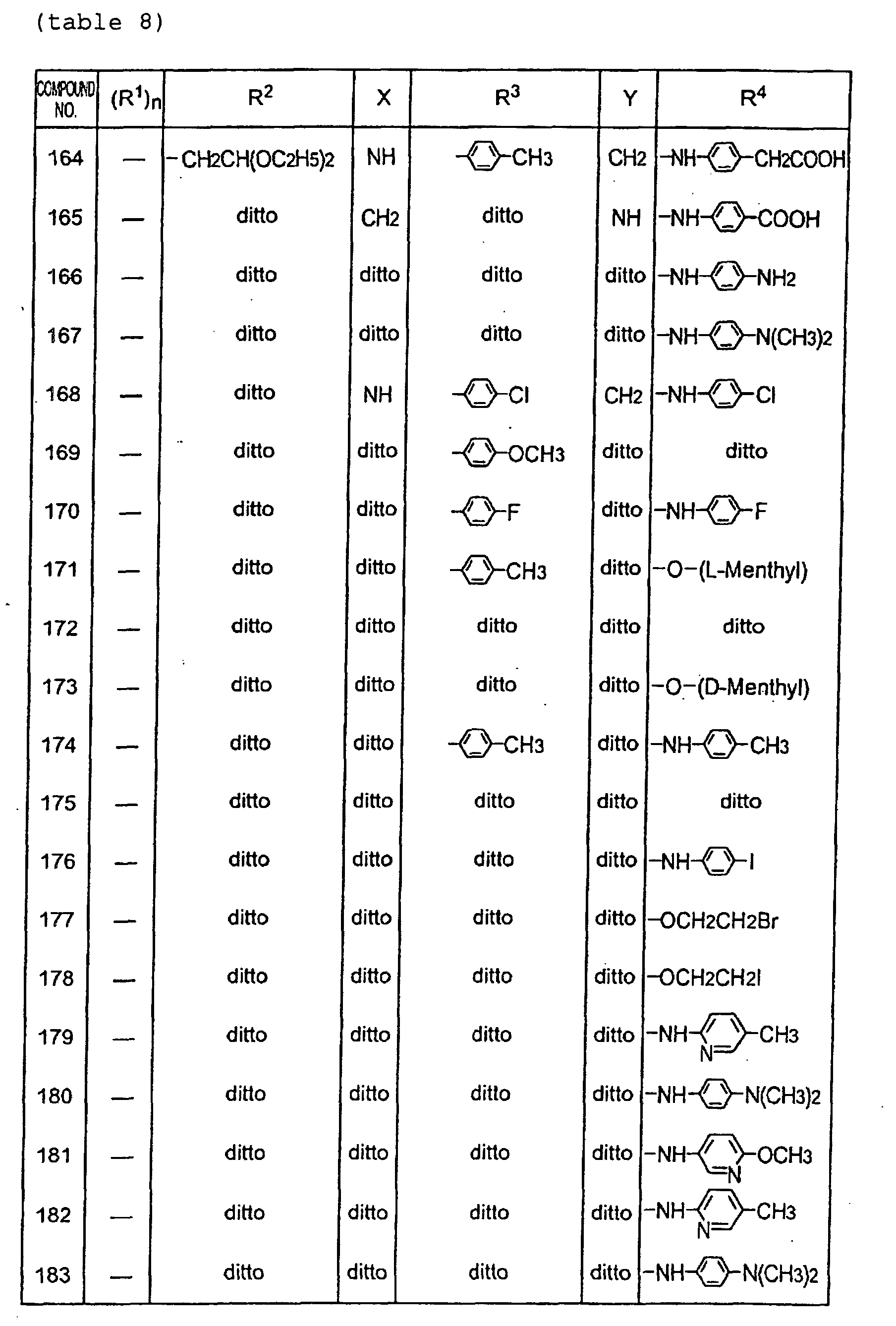

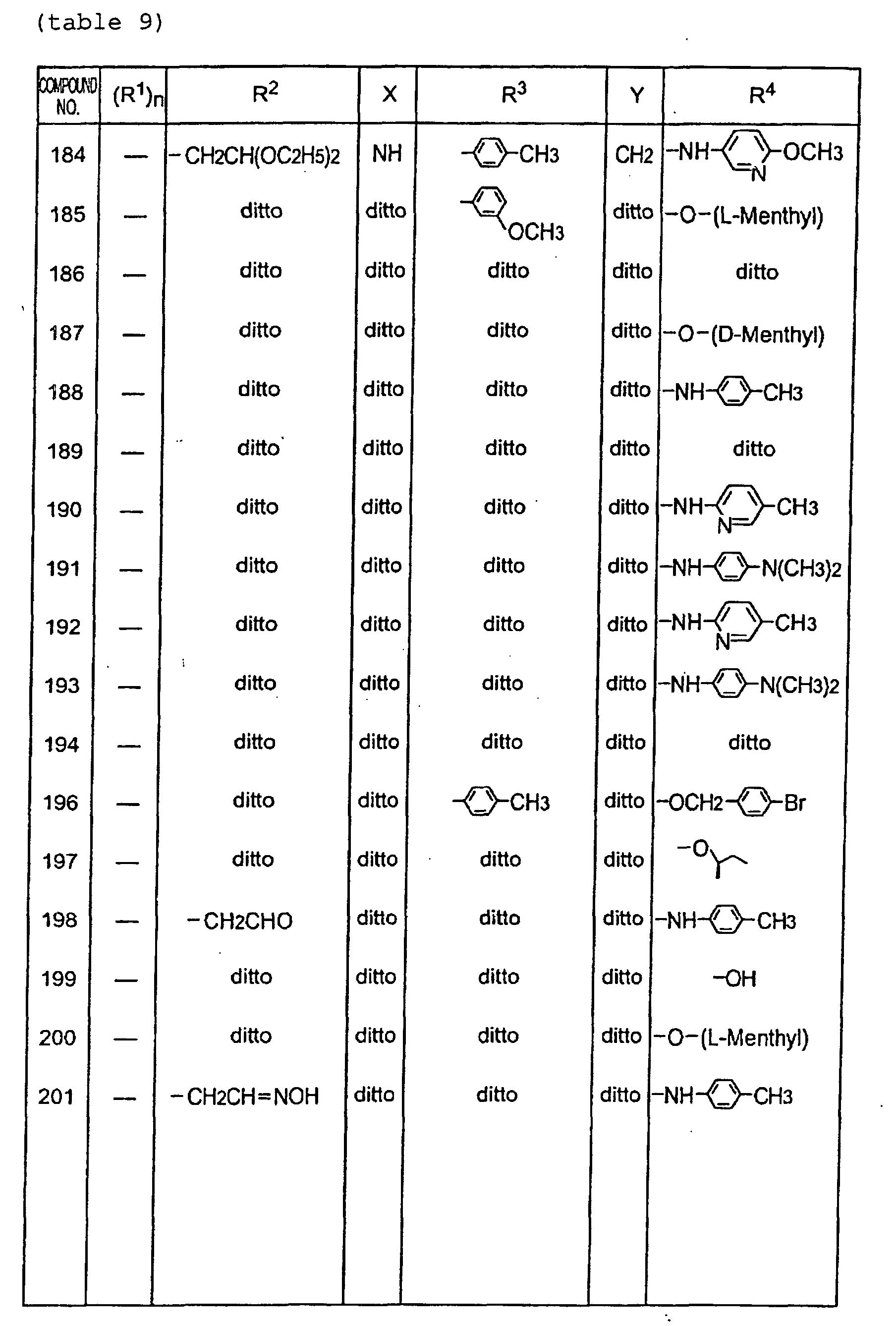

As specific examples of more preferred compounds of indolin-2-one derivatives used for the invention there may be mentioned the compounds represented by the following formulas (A), (B), (C), (D), (E), (F) and (G). These compounds represented by formulas (A), (B), (C), (D), (E), (F) and (G) will hereinafter be referred to as Compounds A, B, C, D, E, F and G, respectively.

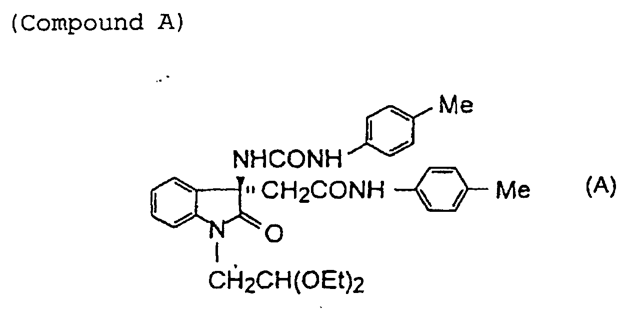

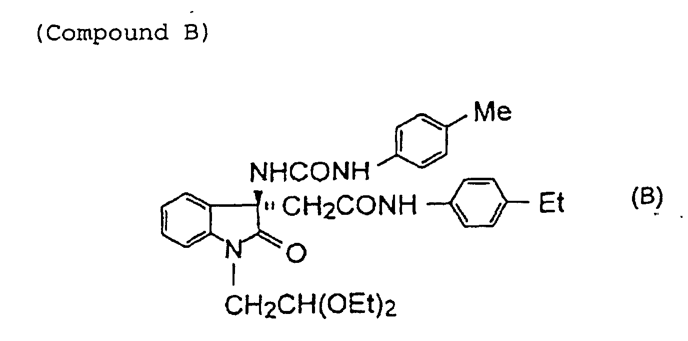

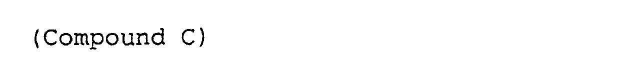

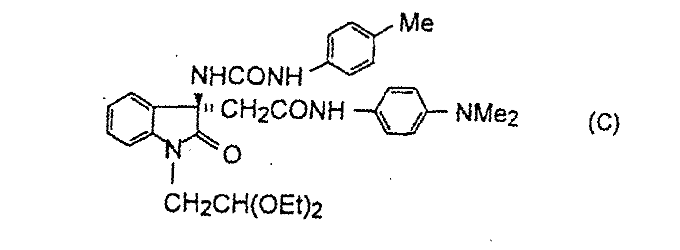

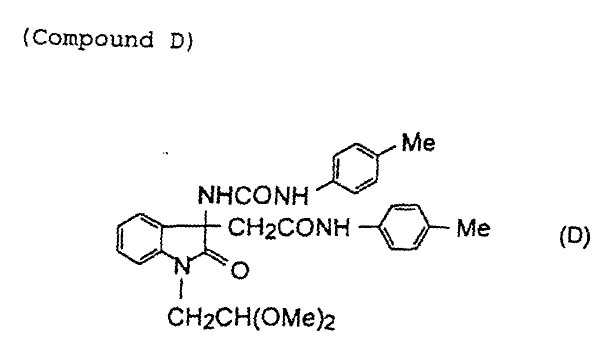

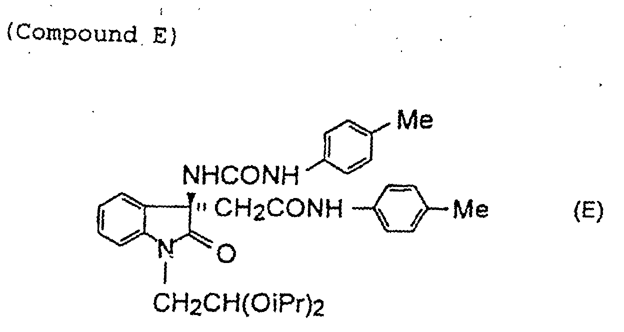

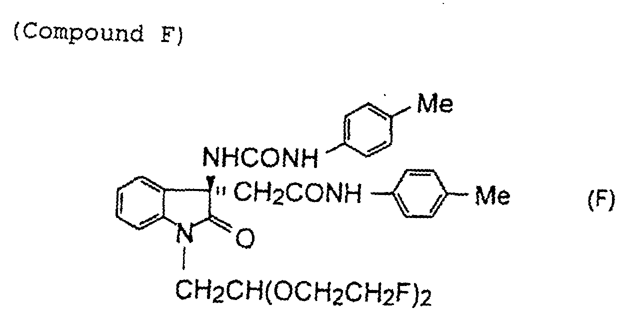

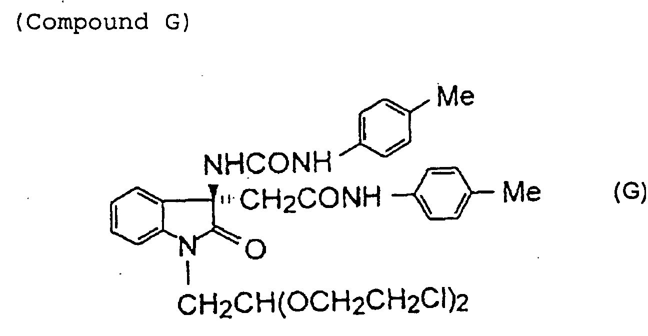

As examples of *in vivo* metabolites of Compound A there may be mentioned the compounds represented by the following formulas (H) and (I). The compounds represented by formulas (H) and (I) will hereinafter be referred to as Compounds H and I, respectively.

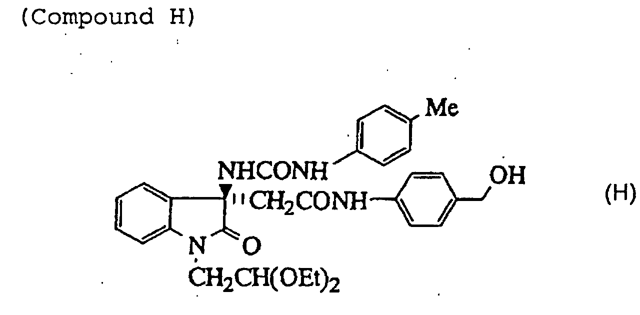

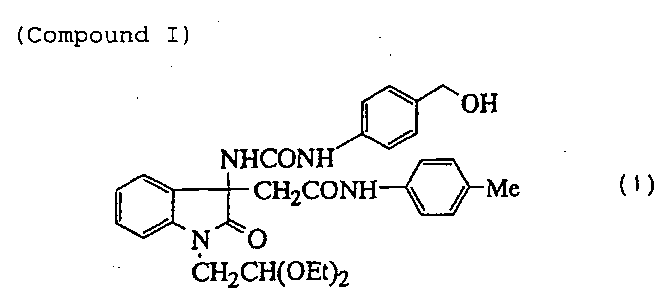

The compounds listed in Tables 1 to 9 may be synthesized by the process described in Japanese Patent Application Laid-Open No. HEI 7-48349. Of the compounds A to G mentioned above, compounds A to D may be synthesized by the process described in Japanese Patent Application Laid-Open No. HEI 7-48349.

Compounds E, F and G may be synthesized, for example, by Reaction Path A shown below (corresponding to "Reaction Path 6" in Japanese Patent Application Laid-Open No. HEI 7-48349), using as the starting material the aldehyde intermediate mentioned in the examples of the present application, synthesized according to the process described in Japanese Patent Application Laid-Open No. HEI 7-48349.

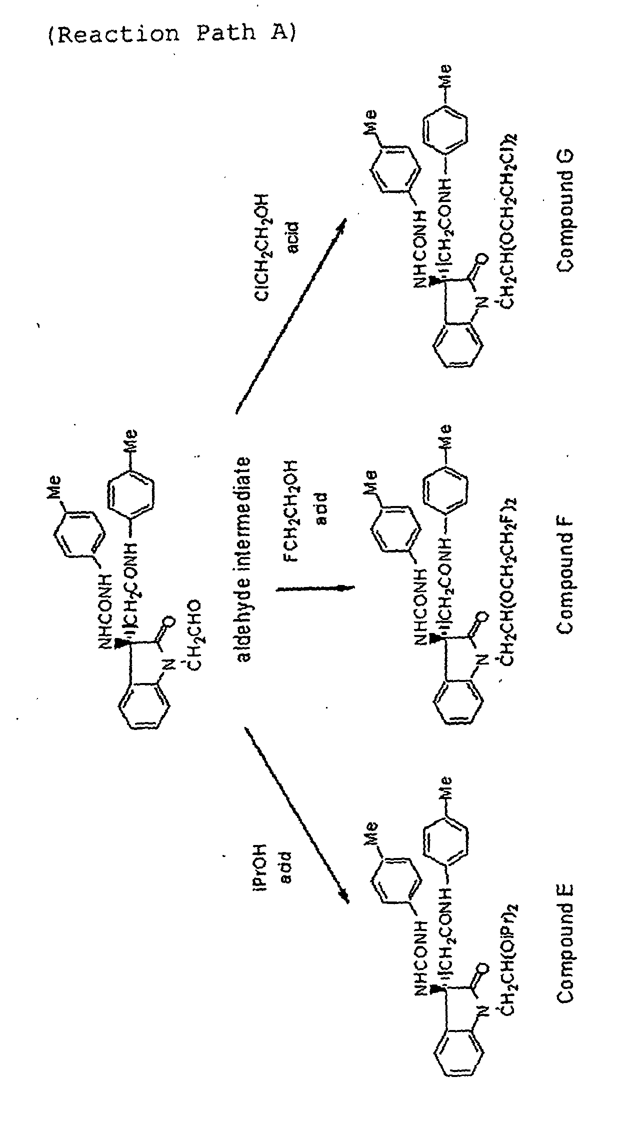

Compound H mentioned above may be synthesized according to "Reaction Path 7" in Japanese Patent Application Laid-Open No. HEI 7-48349. Compound I may be obtained by the following process, either in a racemic form or optically active form.

Specifically, a racemic form of compound I may be synthesized, for example, according to the following Reaction Path B (corresponding to "Reaction Path 5" in Japanese Patent Application Laid-Open No. HEI 7-48349), using an isocyanate having a hydroxyl group protected with a suitable protecting group (for example, a substituted silyl group such as a triethylsilyl group, a t-butyldimethylsilyl group or the like).

An optically active form of compound I may be synthesized, for example, according to the following Reaction Path B (corresponding to "Reaction Path 7" in Japanese Patent Application Laid-Open No. HEI 7-48349), using an isocyanate having a hydroxyl group protected with a suitable protecting group (for example, a substituted silyl group such as a triethylsilyl group, a t-butyldimethylsilyl group or the like), or by optically separating a stereoisomeric mixture of compound I by a method well known to those skilled in the art (for example, a method using an optically active column).

Identification, structure determination and purity determination of the obtained compound may be accomplished by ordinary methods (spectroscopic methods such as NMR, IR, etc. and high performance liquid chromatography or the like).

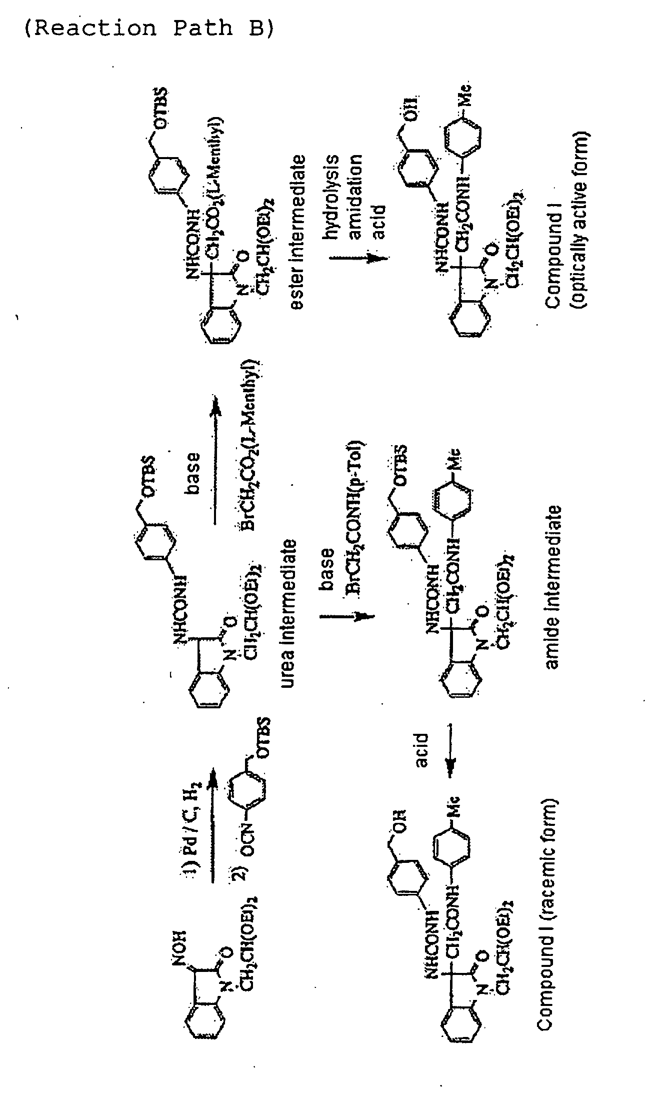

### (Biodegradable and/or biocorrosive polymer)

The biodegradable and/or biocorrosive polymer used for the invention will now be explained.

The polymer used for the invention is biodegradable and/or biocorrosive, and according to the invention, "biodegradable" refers to the property whereby intermolecular and/or intramolecular bonds are broken by biological enzymes (for example, proteases or esterases) or water, while "biocorrosive" refers to the property whereby an insoluble substance is corroded by chemical reaction (chemical reaction by enzymes or water) or physical action (uptake by phagocytes or the like) in vivo.

The biodegradable and/or biocorrosive polymer is a polymer which is capable of encapsulating the drug having a chondrogenesis-promoting action, and is preferably a polymer by which the drug is supported, to form a loaded-structure. Because of their biodegradable and/or biocorrosive properties, such polymers are gradually decomposed (low molecularized) and/or corroded *in vivo,* and thus release their drugs gradually into the body. Consequently, the formulation for cartilage tissue formation of the invention can be a controlled-release preparation.

The loaded-structure may be in any desired form, but in order to control the drug loaded dose and release time, it is preferably in the form of a microsphere, a film or nonwoven fabric. As an example of a method of forming microspheres there may be mentioned solvent evaporation method of an O/W emulsion. As an example of a film-forming method there may be mentioned casting, and as an example of a nonwoven fabric-forming method there may be mentioned a method of formation into a sponge by freeze, followed by compression with a press and creation of a felt. The amount of the drug having a chondrogenesis-promoting action which is supported (encapsulated) by the loaded-structure is determined by the required release dose and the release time of the drug, but it is preferably 1-60% wt/wt and more preferably 5-40% wt/wt. The drug release time and release pattern may be controlled by varying the biodegradable and/or biocorrosive properties of the polymer. Specifically, they may be controlled by varying the size of the shaped polymer (or the particle size in the case of microspheres), the polymerization ratio of the copolymer and the porosity of the polymer.

The drug release property may be controlled by varying the type and molecular weight of the polymer used. Specifically, a higher biodegradable and/or biocorrosive property of the polymer results in a faster drug release rate, while a higher molecular weight of the polymer results in greater hydrophobicity and thus reduced water permeability or a reduced biodegradable and/or biocorrosive property, and therefore a slower drug release rate.

Considering the release time and release pattern of the drug and the biodegradable and/or biocorrosive properties of the polymer, the biodegradable and/or biocorrosive polymer preferably has a weight-average molecular weight of 500 or greater and more preferably a weight-average molecular weight of 500-200,000. More preferably, the polymer has a weight-average molecular weight of 1000 to 100,000.

As biodegradable and/or biocorrosive polymers there may be mentioned polyhydroxyalkanoic acids, lactone ring-opened polymers, hydroxyalkanoic acid/lactone copolymers, polyacetals, polyketals, polymethylvinyl ethers, chitin, chitosan, polyamides, polyamino acids, polyurethanes, polyester amides, polycarboxylic acids, polyanhydrides, polyalkylenes, polyphosphoric acids, polyorthoesters, and copolymers comprising one or more of the foregoing in the molecule. These polymers may be used alone or in combinations of two or more.

As polyhydroxyalkanoic acids there may be mentioned hydroxyalkanoic acid polymers such as glycolic acid polymers (polyglycolic acid), lactic acid polymers (polylactic acid), hydroxybutyric acid polymers (polyhydroxybutyric acid), hydroxyvaleric acid polymers (polyhydroxyvaleric acid) and the like. As copolymers of hydroxyalkanoic acids there may be mentioned lactic acid/glycolic acid copolymer, hydroxybutyric/hydroxyvaleric acid copolymer and the like. Incidentally, the polyhydroxyalkanoic acids and hydroxyalkanoic acids may also have substituents, and preferably have no more than 12 carbon atoms. According to the invention, lactic acid/glycolic acid copolymer is particularly preferred.

As lactone ring-opened polymers there may be mentioned butyrolactone ring-opened polymers (polybutyrolactone), valerolactone ring-opened polymers (polyvalerolactone), caprolactone ring-opened polymers (polycaprolactone), dioxanone ring-opened polymers (polydioxanone) and the like, and as hydroxyalkanoic acid/lactone copolymers there may be mentioned copolymers of the aforementioned hydroxyalkanoic acids and lactones, such as glycolic acid/lactone copolymers and lactic acid/valerolactone copolymers.

Polyamides to be used include biodegradable and/or biocorrosive reaction products of polyamines and polycarboxylic acids, and preferably there may be mentioned reaction products of aliphatic polyamines of no greater than 12 carbon atoms and aliphatic polycarboxylic acids of no greater than 12 carbon atoms Polyamino acids are polymers of aminocarboxylic acids (amino acids), and include oligopeptides and polypeptides (proteins).

Polyurethanes are reaction products of polyols and polyisocyanates and are not particularly restricted so long as they are biodegradable and/or biocorrosive, but reaction products of aliphatic polyols of no greater than 12 carbon atoms and aliphatic isocyanates of no greater than 12 carbon atoms are preferred.

Polyester amides to be used are biodegradable and/or biocorrosive compounds having both ester bonds and amide bonds in the molecule, and preferably there may be mentioned compounds having a polyester backbone composed of an aliphatic polyol of no greater than 12 carbon atoms and an aliphatic polycarboxylic acid of no greater than 12 carbon atoms, and a polyamide backbone composed of an aliphatic polyamine of no greater than 12 carbon atoms and an aliphatic polycarboxylic acid of no greater than 12 carbon atoms.

As polycarboxylic acids there may be mentioned polymers of compounds comprising two or more carboxyl groups, such as polymaleic acid, polymalic acid and polyglutamic acid, and as polyanhydrides there may be mentioned polymers of compounds having a carboxylic anhydride structure, such as polysebacic anhydride and polymaleic anhydride. As polyalkylenes there may be mentioned polymers having an alkylene group and an ester group, such as polyalkyleneoxalates and polyalkylenesuccinicates.

As polyphosphoric acids there may be mentioned linear polymers produced by dehydration of orthophosphoric acids, and as polyorthoesters there may be mentioned 3,9-bis(ethylidene-2,4,8,10-tetraoxaspiro[5.5]undecane based polymer.

As part of the polymers of the invention, reactive polymers containing unsaturated double bonds may be used. Reactive polymers containing unsaturated double bonds polymerize by mixing with polymerization catalysts or giving heat or light energy. In case of using reactive polymers containing two or more unsaturated double bonds, three dimensional crosslinks are formed by the polymerization.

As preferable reactive polymers, there may be mentioned compounds comprising a backbone and one or more (meth)acryloyl group bonding to the backbone, wherein the backbone comprising one or more polymers selected from a group consisting of polyhydroxyalkanoic acids, lactone ring-opened polymers, hydroxyalkanoic acid/lactone copolymers, polyacetals, polyketals, polymethylvinyl ethers, chitin, chitosan, polyamides, polyamino acids, polyurethanes, polyester amides, polycarboxylic acids, polyanhydrides, polyalkylenes, polyphosphoric acids, polyorthoesters, and copolymers comprising one or more of the foregoing in the molecule. (Meth)acryloyl groups represent acryloyl groups or methacryloyl groups.

As Polyhydroxyalkanoic acids, lactone ring-opened polymers, hydroxyalkanoic acid/lactone copolymers, polyacetals, polyketals, polymethylvinyl ethers, chitin, chitosan, polyamides, polyamino acids, polyurethanes, polyester amides, polycarboxylic acids, polyanhydrides, polyalkylenes, polyphosphoric acids and polyorthoesters, which are backbones of reactive polymers, may be used the similar compounds mentioned above.

Although the binding site and the number of (meth)acryloyl groups bonding to the backbone are not particularly restricted, preferably, (meth)acryloyl groups bond to one or more terminal of the backbone.

In case of (meth) acryloyl groups bond to the terminals of polyesters such as polyhydroxyalkanoic acids, lactone ring-opened polymers and hydroxyalkanoic acid/lactone copolymers, (meth)acryloyloxy bonds ((meth)acrylate bonds) can form.

When the polymer used in the formulation for cartilage tissue formation contains one of the aforementioned reactive polymers, a polymerization initiator for the reactive polymer is preferably added to the formulation for cartilage tissue formation before use. The polymerization initiator is preferably added immediately prior to use from the standpoint of stability of the formulation for cartilage tissue formation with time. The polymerization initiator used is not particularly restricted, and as examples there may be mentioned azo compounds such as azobisisobutyronitrile (AIBN), peroxides such as benzoyl peroxide, two-component redox initiators such as benzoyl peroxide-dimethylaniline, and photoinitiators such as α,α-dimethoxy-α-phenylacetophenone (DMPA).

Since the formulation for cartilage tissue formation of the invention is to be used *in vivo,* the polymerization initiator is preferably one which is catalytically active at near body temperature or below body temperature.

### (Porous matrix and hydrogel which substantially do not inhibit cartilage tissue repair)

The following explanation will describe the porous matrix and hydrogel which substantially do not inhibit cartilage tissue repair.

The formulation for cartilage tissue formation of the invention comprises the aforementioned drug and polymer, with a porous matrix which substantially does not inhibit cartilage tissue repair and/or a hydrogel which substantially does not inhibit cartilage repair, and such a porous matrix and hydrogel are scaffold-forming materials which function as scaffolding for formation of cartilage tissue *in vivo.* However, since retention of the scaffold *in vivo* (joints, etc.) for long periods can instead prevent repair of the cartilage tissue, the porous matrix or hydrogel must be a material which substantially does not inhibit cartilage tissue repair.

The drug release property of the formulation for cartilage tissue formation of the invention in vivo may be controlled by varying the type and combination of the drug and polymer contained therein, but independently of these factors, the scaffold-forming function for cartilage tissue repair may also be adjusted by varying the composition of the porous matrix and/or hydrogel contained therein. Thus, since the drug release property and scaffold-forming property for cartilage tissue repair can be independently controlled, it is possible to achieve efficient and stable repair of cartilage tissue as compared to compositions which contain a drug and polymer but contain no porous matrix or hydrogel.

When a porous matrix is used as the scaffold for cartilage tissue repair, the porous matrix preferably has a pore size (diameter) of sufficient size to allow infiltration of chondrocytes, and the mean pore size is therefore preferably 10-500 µm.

Since the porous matrix can inhibit repair of cartilage tissue if it resides in vivo (joints) for long periods, as mentioned above, it is preferably decomposed and/or corroded *in vivo* and eliminated appropriately within a relatively short time. That is, the porous matrix preferably consists of a biodegradable and/or biocorrosive compound. The in vivo elimination rate is preferably 1 day to 5 weeks and more preferably 1 to 4 weeks. The porous matrix preferably consists of a compound with low toxicity and antigenicity, preferably having a weight-average molecular weight of 500 or greater.

Biodegradable and/or biocorrosive compounds to be used as porous matrices include the same polymers as the biodegradable and/or biocorrosive polymers mentioned above as constituents except the porous matrix. Specifically, there may be used polyhydroxyalkanoic acids, lactone ring-opened polymers, hydroxyalkanoic acid/lactone copolymers, polyacetals, polyketals, polymethylvinyl ethers, chitin, chitosan, polyamides, polyamino acids, polyurethanes, polyester amides, polycarboxylic acids, polyanhydrides, polyalkylenes, polyphosphoric acids, polyorthoesters, and copolymers comprising one or more of the foregoing in the molecule, among which polymers or copolymers of compounds selected from the group consisting of glycolic acid, lactic acid, hydroxybutyric acid and hydroxyvaleric acid are preferred, and lactic acid/glycolic acid copolymer is particularly preferred.

At least a part of the biodegradable and/or biocorrosive polymer used as the porous matrix may be a reactive polymer having an unsaturated double bond. Specific examples thereof include the same biodegradable and/or biocorrosive polymers mentioned above as constituents except the porous matrix.

As porous matrices which substantially do not inhibit cartilage repair there may be preferably used matrices comprising hyaluronic acid derivatives or collagen. As hyaluronic acid derivatives there may be mentioned physically crosslinked hyaluronic acid and chemically crosslinked hyaluronic acid with low-crosslinked density (for example, with a crosslinked density of 10% or lower), among which there are preferred hydrogen bond-type physically crosslinked hyaluronic acid or ionic bond-type physically crosslinked hyaluronic acid. Preferred examples of collagen include collagen I and II.

Particularly preferred as biodegradable and/or biocorrosive compounds to be used as porous matrices are lactic acid/glycolic acid copolymer, hydrogen bond-type physically crosslinked hyaluronic acid, ionic bond-type physically crosslinked hyaluronic acid, and collagen.

There are no particular restrictions on the method of forming the porous matrix, and for example, there may be mentioned a method of freeze of a dilute solution or a gel, and a method of molding in the copresence of a filler and dissolving and extracting the filler..

According to the invention, a hydrogel which substantially does not inhibit cartilage repair may be used either in place of the porous matrix, or together with the porous matrix. Such a hydrogel is a material which gels upon swelling by water or body fluids, and it functions as a scaffold for cartilage tissue repair, in a manner similar to the porous matrix described above.

The hydrogel may be gelled *in situ*, and as an example of a method of gelling *in situ* there may be mentioned a method of injecting the formulation for cartilage tissue formation, comprising the hydrogel at a non-gelled state (or at a fluid gel state) into the cartilage defect lesion using a syringe or the like. In this case, gelling of the hydrogel occurs at the injected portions, thereby forming a scaffold for cartilage tissue repair. That is, by using the hydrogel as a scaffold for cartilage tissue repair, it is possible to facilitate injection and scaffold formation and thereby expedite application of the formulation for cartilage tissue formation.

Since the hydrogel can inhibit repair of cartilage tissue if it resides *in vivo* (joints) for long periods, similar to a porous matrix, it is preferably decomposed and/or corroded *in vivo* and eliminated appropriately within a relatively short time. That is, the hydrogel preferably consists of a biodegradable and/or biocorrosive compound. The *in vivo* elimination rate is preferably 1 day to 5 weeks and more preferably 1 to 4 weeks. The hydrogel preferably consists of a compound with low toxicity and antigenicity, preferably having a weight-average molecular weight of 500 or greater.

Suitable biodegradable and/or biocorrosive compounds to be used as hydrogels include the same biodegradable and/or biocorrosive compounds mentioned above as examples for the porous matrix. In order to increase the efficiency of gelling *in situ*, a method may be employed wherein a polymerizable functional group such as vinyl is introduced into the biodegradable and/or biocorrosive compound and the compound is injected into the cartilage defect lesion together with a polymerization initiator which generates radicals by light or heat, and then solidified by light- or heat-induced crosslinking (J. Stephanie et al., Biomaterials 22 619 (2001), J. Stephanie et al., J. Biomater. Sci. Polym. Ed., 11, 439 (2000), and other publications). Alternative methods to be employed include a method of injecting into the defect lesion a hydrogel material having a light-crosslinking photoreactive functional group such as cinnamate introduced therein, and then solidifying it by light-induced crosslinking (Japanese Patent Application Laid-Open No. HEI 6-73102, J. Polym. Sci. Polym. Chem. 30, 2451 (1992), and other publications), and a method of solidifying (gelling) by physical crosslinking based on temperature increase by body temperature after injection (BST-Gel™ (Biosyntech), ReGel™ (Macromed), etc.). In order to limit the *in vivo* elimination rate of the hydrogel to between 1 day and 5 weeks (preferably 1 to 4 weeks), the introduction of crosslinking functional groups in the hydrogel is preferably low and the crosslinking density of the hydrogel is preferably low (for example, 10% or less).

### (Formulation for cartilage tissue formation)

The formulation for cartilage tissue formation according to the invention will now be explained. The formulation for cartilage tissue formation of the invention need only comprise a drug having a chondrogenesis-promoting action, a biodegradable and/or biocorrosive polymer, and a porous matrix which substantially does not inhibit cartilage tissue repair and/or a hydrogel which substantially does not inhibit cartilage repair. Here, the drug and polymer form a loaded-structure with the drug supported by the polymer, while the porous matrix and/or hydrogel are preferably in contact with the loaded-structure.

There are no particular restrictions on the method of producing the formulation for cartilage tissue formation, and it may involve merely appropriate mixture of the drug which promotes cartilage tissue formation, the biodegradable and/or biocorrosive polymer and the porous matrix which substantially does not inhibit cartilage tissue repair and/or the hydrogel which substantially does not inhibit cartilage repair.

When a porous matrix which substantially does not inhibit cartilage tissue repair is used as the scaffold for cartilage tissue repair, the formulation for cartilage tissue formation may be prepared by the following method. For example, when the loaded-structure comprising the drug and polymer is in the form of microspheres, the microspheres may be dispersed in a solvent together with the matrix material (the material serving as the porous matrix) and the matrix then rendered porous by a method of freeze, filler extraction or the like, to produce the formulation for cartilage tissue formation. When the loaded-structure is in the form of a film or nonwoven fabric, the same method may be applied as for microspheres, but there may alternatively be applied a method wherein the matrix is rendered porous by freeze, filler extraction or the like, and then a loaded-structure in the form of a film or nonwoven fabric is anchored therein. Anchoring of the porous matrix when the loaded-structure is in the form of a film or nonwoven fabric may be accomplished either before implanting, if the formulation for cartilage tissue formation is to be applied by implanting in the cartilage defect lesion, or the loaded-structure may be anchored after the porous matrix has been implanted in the defect lesion. In the latter case, the loaded-structure is preferably anchored at a position distant from the initiating site of cartilage regeneration so that the biodegradable and/or biocorrosive polymer does not inhibit cartilage tissue regeneration.

When a hydrogel which substantially does not inhibit cartilage tissue repair is used as the scaffold for cartilage tissue repair, the formulation for cartilage tissue formation may be prepared by the following method. Specifically, when the drug and polymer form a loaded-structure and the loaded-structure is in the form of microspheres, the microspheres may be dispersed in the hydrogel before gelling. A formulation prepared by this method is injected *in situ* for gelling of the hydrogel. The gelling may be before in situ injection if fluidity of the hydrogel can be ensured. When the loaded-structure is in the form of a film or nonwoven fabric, the same method may be applied as for microspheres, but there may alternatively be applied a method wherein the hydrogel is gelled and then the film or nonwoven fabric loaded-structure is anchored therein. Anchoring of the hydrogel when the loaded-structure is in the form of a film or nonwoven fabric may be accomplished either before implanting, if the formulation for cartilage tissue formation is to be applied by implanting in the cartilage defect lesion, or the loaded-structure may be anchored after the gelled hydrogel has been implanted in the defect lesion (or after the hydrogel has gelled at the defect lesion). In the latter case, the loaded-structure is preferably anchored at a position distant from the initiating site of cartilage regeneration so that the biodegradable and/or biocorrosive polymer does not inhibit cartilage tissue regeneration.

According to the invention, the total weight of the drug having a chondrogenesis-promoting action and the biodegradable and/or biocorrosive polymer is preferably 0.1-50 parts by weight and most preferably 0.1-30 parts by weight, with respect to 100 parts by weight as the total of the porous matrix which substantially does not inhibit cartilage tissue repair and/or the hydrogel which substantially does not inhibit cartilage repair.

The formulation for cartilage tissue formation may be used as an implanted formulation which is directly implanted into a cartilage defect lesion, or as a coating formulation which is directly coated at a cartilage defect lesion. Otherwise, it may be used as a formulation for injection which is administered *in vivo* by injection, or as an endoscopic formulation which is administered *in vivo* by endoscope. Use of the formulation in this manner allows it to serve as a treatment agent for *in vivo* repair and regeneration of cartilage of a congenital or traumatic cartilage defect patient. Moreover, culturing of chondrocytes placed in the solidified formulation may be carried out for formation of cartilage *ex vivo,* thereby allowing application as a treatment by formation of cartilage into the necessary shape from autologous chondrocytes and transplantation thereof into the body. The formulation for cartilage tissue formation of the invention therefore has potential application as a formulation for repair, regeneration and formation of cartilage both *in vivo* and *ex vivo* in the field of regenerative medical engineering (tissue engineering), which is currently a subject of intense research.

### (Examples)

Preferred examples of the invention will now be explained in detail with the understanding that the invention is in no way limited by these examples.

### [Example 1] Synthesis of drug having chondrogenesis-promoting action

Compound A described above was synthesized by the method described in "Example 174(2)" of Japanese Patent Application Laid-Open No. HEI 7-48349.

### [Example 2] Preparation of porous matrix or hydrogel which substantially does not inhibit cartilage repair

### (Example 2-1) Preparation of hydrogel with physically crosslinked hyaluronic acid

Hyaluronic acid with a weight-average molecular weight of 1.8 x 10⁶ (Suvenyl™, Chugai Pharmaceutical Co., Ltd.) was dissolved in distilled water to prepare a 2% solution. After then adding 1 N hydrochloric acid to adjust the pH to 1.0, the solution was drawn into a 1 mL syringe and frozen at -20°C for 120 hours. After restoring it to room temperature, it was washed with phosphate-buffered saline (hereinafter, PBS) and allowed to stand in PBS for 24 hours to yield a water-insoluble hydrogel (physically crosslinked HA hydrogel). "HA" represents hyaluronic acid.

### (Example 2-2) Preparation of porous matrix with lactic acid/glycolic acid copolymer

Lactic acid/glycolic acid copolymer (hereinafter, PLGA) (weight-average molecular weight: 95,000, Medisorb™, Alkermes Inc.) was dissolved in 1,4-dioxane (Junsei Chemical Co., Ltd.) (1.0 w/v%), and the solution was drawn into a 1 mL syringe, frozen at -80°C and then dried *in vacuo* to yield a porous matrix (PLGA sponge).

### (Example 2-3) Preparation of porous matrix with collagen I

A 0.3 w/v% solution of collagen I (Cellmatrix I™, Nitta Gelatin Inc.) in HCl (pH 3.0) was drawn into a 1 mL syringe and frozen at -80°C and then dried *in vacuo* to form a sponge which was crosslinked by UV irradiation (UV Cross-Linker CL-1000, Funakoshi Co., Ltd.) to yield a porous matrix (collagen I sponge).

### (Example 2-4) Preparation of porous matrix with collagen II

A 0.3 w/v% solution of collagen II (Cellmatrix II™, Nitta Gelatin Inc.) in HCl (pH 3.0) was drawn into a 1 mL syringe and frozen at -80°C and then dried *in vacuo* to form a sponge which was crosslinked by UV irradiation (UV Cross-Linker CL-1000, Funakoshi Co., Ltd.) to yield a porous matrix (collagen II sponge).

### (Comparative Example 2-1) Preparation of hydrogel with chemically crosslinked hyaluronic acid

To a solution of HA in 0.2 N NaOH (2 w/v%) was added divinylsulfone (hereinafter, DVS) (Aldrich) (HA monomer/DVS = 0.5 wt/wt), and the mixture was drawn into a 1 mL syringe, removed after 1 hour, washed with water and then immersed in PBS to yield a hydrogel (chemically crosslinked HA hydrogel).

### (Comparative Example 2-2) Preparation of porous matrix with chemically crosslinked hyaluronic acid

To a solution of HA in 0.2 N NaOH (2 w/v%) was added DVS (Aldrich) (HA monomer/DVS = 0.5 wt/wt), and the mixture was drawn into a 1 mL syringe, and after 1 hour the gel was removed and washed with water. It was then freezed to yield a porous matrix (chemically crosslinked HA sponge).

### (Test Example 2-1) Confirmation of fine structure of porous matrix by scanning electron microscope

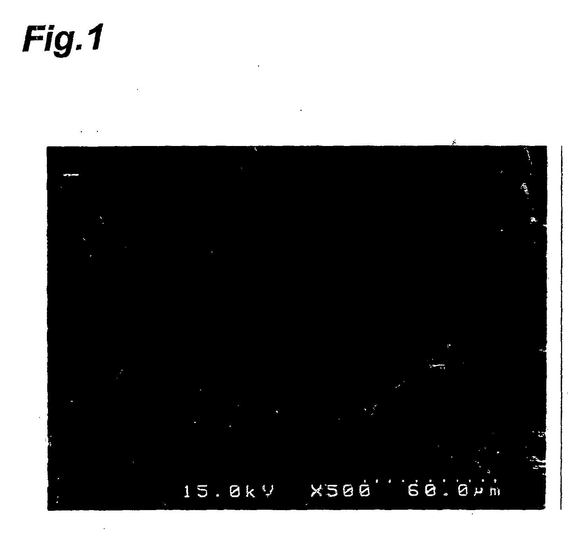

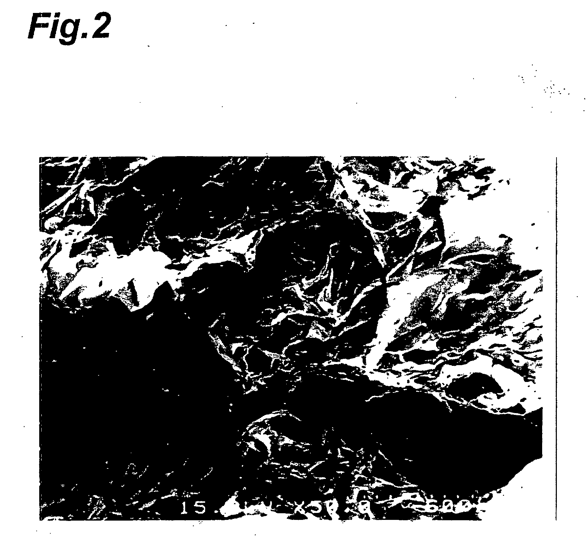

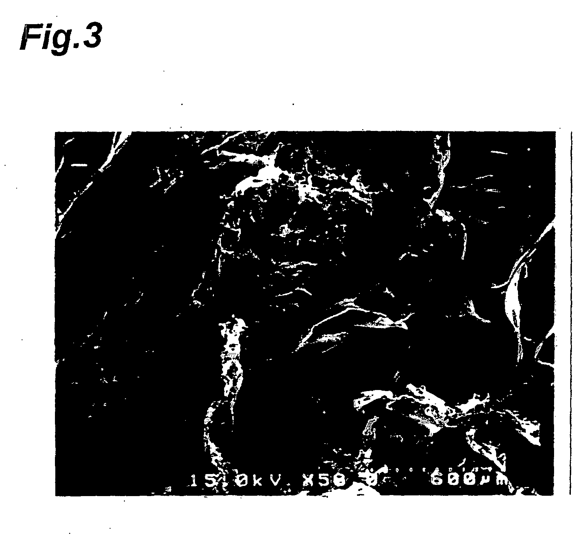

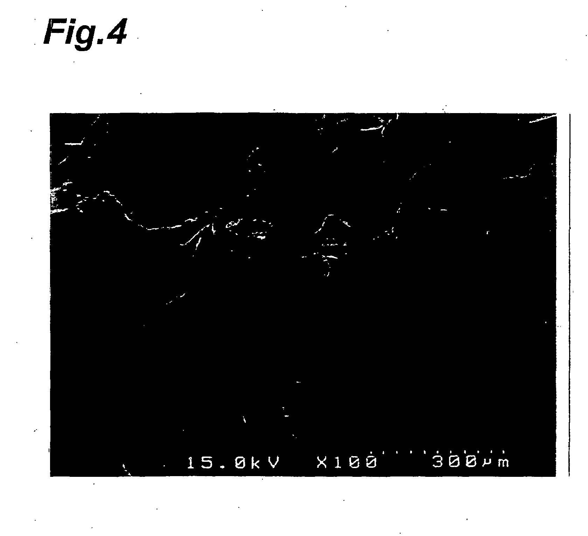

The fine structures of the porous matrices of Examples 2-2 to 2-4 and Comparative Example 2-2 were observed with a scanning electron microscope (SEM) (S-4200, Hitachi). The obtained electron micrographs are shown in Figs. 1 to 4. These confirmed that the PLGA sponges had porous structures with pore sizes of about 10-100 µm. However, the collagen I sponge and collagen II sponge had side-partitioned porous structures with pore sizes of about 100-600 µm, while the chemically crosslinked HA sponge had a side-partitioned porous structure with pore sizes of about 100-300 µm.

### (Test Example 2-2) Proliferation of rabbit bone marrow-derived mesenchymal stem cells on porous matrix and hydrogel substrates

One 3-week-old male Japanese white rabbit (Kitayama Labes Co., Ltd.) was euthanized, and then the femur and tibia were extirpated. After opening a hole in each knee joint side, the diaphysis on the opposite side was cut off. An 18G injection needle was inserted from the knee joint side and the marrow was washed with washing medium (Dulbecco's Modified Eagle's Medium (high glucose) (Nikken Seibutsu Igaku Laboratories) containing 6000 units/mL heparin (Sigma) and 50 units/mL penicillin/50 µg/mL streptomycin (Gibco)). The washing medium containing the marrow cells was centrifuged at 1000 rpm for 5 minutes to precipitate the cells. The obtained marrow cells were seeded on a 10 cm culturing plate (Falcon) and cultured in Dulbecco's Modified Eagle's Medium (high glucose) (Nikken Seibutsu Igaku Laboratories) containing 10% fetal bovine serum (Intergen) and an antibiotic-antifungal agent (Gibco). On the third day, the medium was changed and the non-adherent cells were removed. From the fifth day, 1 ng/mL of basic fibroblast growth factor (bFGF) (Biomedical Technologies) was added to the medium. The medium was then changed every 2-3 days thereafter. On the 14th day, the mesenchymal stem cells which had proliferated into colonies were collected with Trypsin/EDTA (Sigma) and reseeded at a cell density of 5000 cell/cm². Upon reaching approximate confluency, the cells were collected with Trypsin/EDTA (Sigma) and freezed using a Cell Banker (Dia-Iatron) for use in the following experiment.

The rabbit bone marrow-derived mesenchymal stem cells were cultured and collected with Trypsin/EDTA (Sigma) to prepare a cell suspension of 10⁷ cell/mL. A 10 µL portion of the cell suspension (10⁵ cells) was seeded in the porous matrix or hydrogel substrate (Examples 2-2 to 2-4, Comparative Examples 2-1 to 2-2) preshaped into a cylinder with a diameter of 5 mm and a length of 7 mm, and allowed to stand at 37°C for 2 hours and 30 minutes for adsorption of the cells. After cell adsorption, Dulbecco's Modified Eagle's Medium (high glucose) (Nikken Seibutsu Igaku Laboratories) containing 1 ng/mL of basic fibroblast growth factor (bFGF) (Biomedical Technologies), 10% fetal bovine serum (Intergen) and an antibiotic-antifungal agent (Gibco) was added, and culturing was initiated on the porous matrix or hydrogel substrate. The medium was changed every 2-3 days. On the 7th, 14th and 21st days after the start of culturing, the porous matrix or hydrogel (scaffold) was recovered and the cultured cells were disrupted by sonication in distilled water. After centrifugation at 10,000 rpm for 5 minutes, the insoluble portion of porous matrix or hydrogel fragments were removed and the DNA content in the supernatant was measured using a FluoReporter Blue Fluorometric dsDNA Quantification Kit (Molecular Probes).

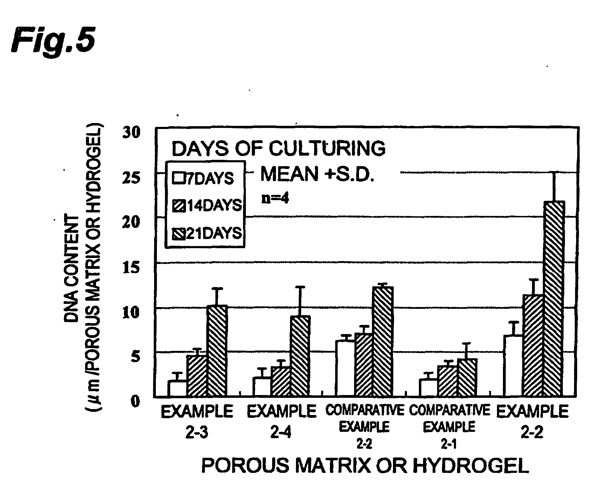

The DNA contents for each porous matrix and hydrogel are shown in Fig. 5. Proliferation of rabbit bone marrow-derived mesenchymal stem cells was found in all of the porous matrices and hydrogel substrates.

### (Test Example 2-3) Compatibility of porous matrix or hydrogel with cartilage tissue in rabbit articular cartilage defect models

The porous matrices or hydrogels of Examples 2-1 to 2-4 and Comparative Examples 2-1 to 2-2 were cut into 4 mm-long cylindrical shapes and used for an in vivo test. of compatibility of each porous matrix or hydrogel with cartilage tissue in rabbit articular cartilage defect models. Using 12-week-old male Kbl:JW rabbits under pentobarbital (Dynabot) anesthesia (40 mg/kg, i.v.), an incision was made in the right articular capsule to expose the femur patellar surface, and a hand drill and 5 mm-diameter metal drill blade (Kobelco) were used to create a defect lesion reaching a 4 mm depth in the bone marrow. The 4 mm-long porous matrix or hydrogel was implanted for one month, and then the articular capsule and skin were sutured. Defect lesions alone were created in individual rabbits as a control. Four weeks after defect creation and implantation of the porous matrix or hydrogel, the rabbits were systemically bled under pentobarbital anesthesia for euthanasia, and the right femurs containing the defect lesions were removed and immersed for one week in 20% neutral buffered formalin (Wako Pure Chemical Industries Co., Ltd.) for fixation. After transecting the center of each defect lesion with a band saw (Exakt), it was demineralized for one week in 5% formic acid (Wako Pure Chemical Industries Co., Ltd.). This was followed by neutralization overnight with a 20% sodium acetate solution and preparation of paraffin sections according to an ordinary protocol. The sections were stained with Safranin O-Fast Green and observed with an optical microscope (Nikon) for histological evaluation of subchondral bone repair in the defect lesions.

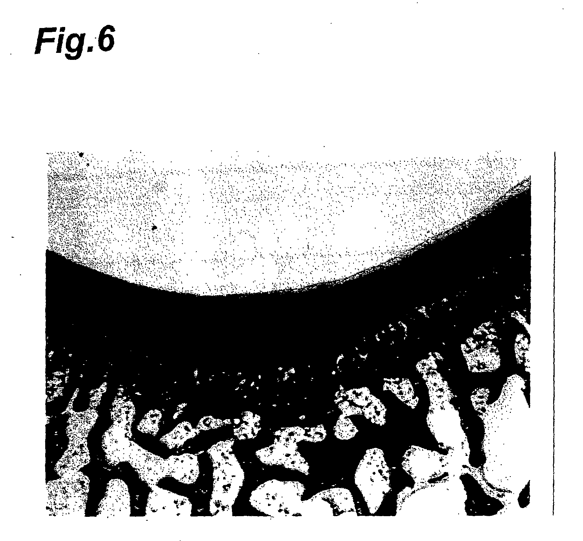

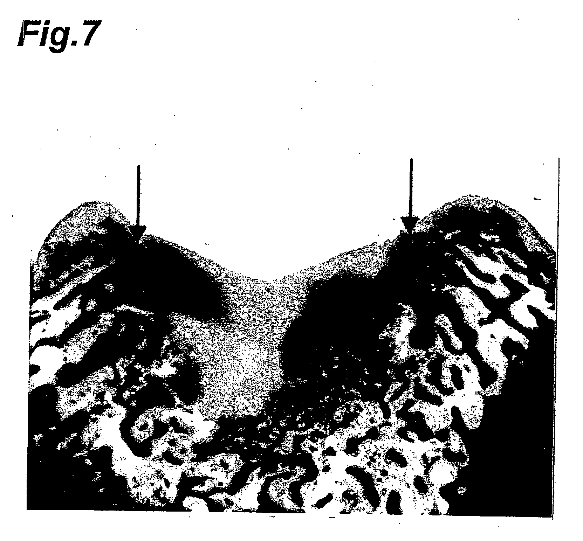

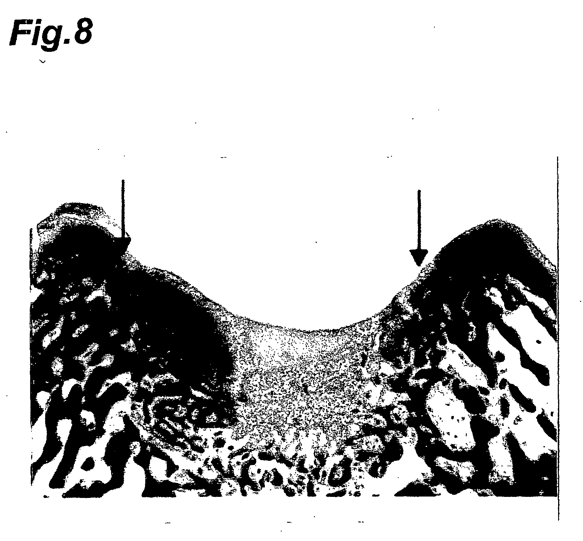

The results for the tissue samples are shown in Figs. 6 to 11. Fig. 6 shows normal tissue with no articular cartilage defect. Fig. 7 shows tissue from an individual having only a defect lesion. Figs. 8, 9, 10 and 11 shows the results upon implanting the porous matrices or hydrogels of Example 2-1, Example 2-2, Comparative Example 2-1 and Comparative Example 2-2, respectively. In these photographs, the areas between the arrows indicate the defect lesions (with the arrows indicating the borders between the defect lesions and normal sites).

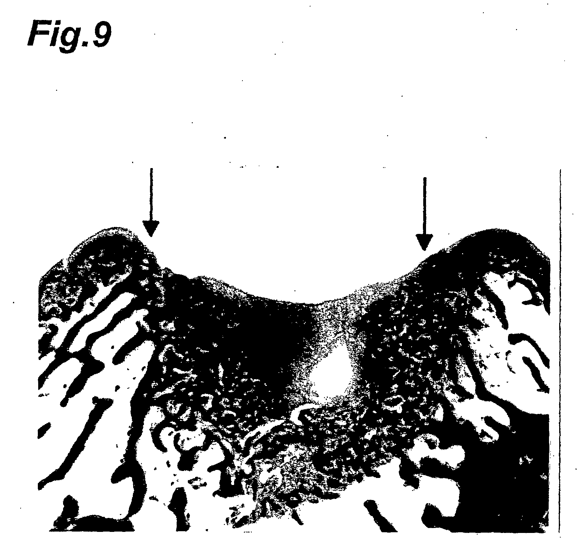

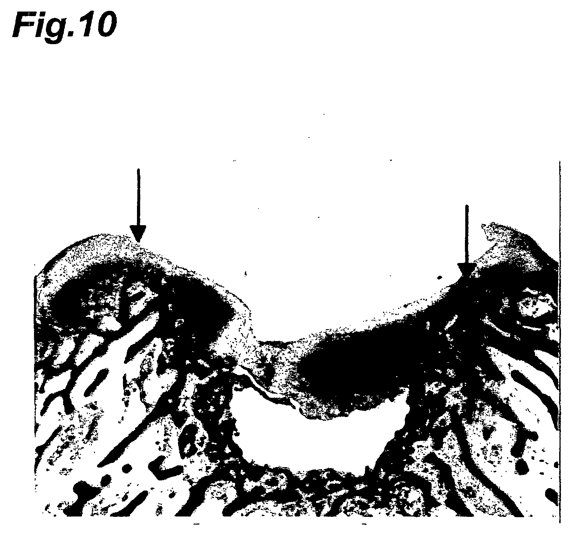

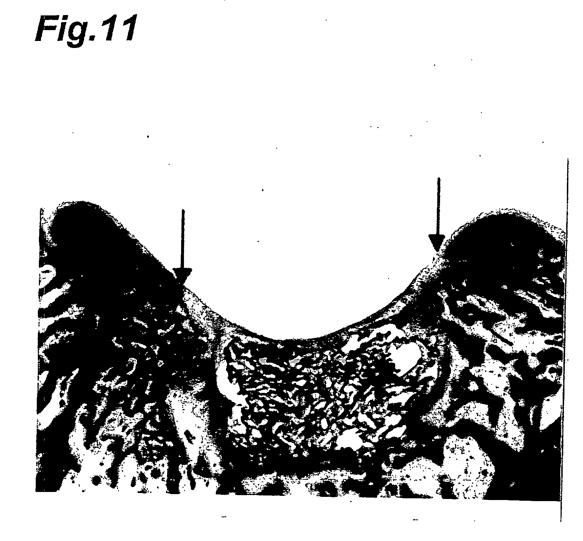

Figs. 6 to 11 clearly show that when the hydrogel of Example 2-1 (Fig. 8) or the porous matrix of Example 2-2 (Fig. 9) was used, the porous matrix was eliminated from the deficient site and natural repair of the subchondral bone at the deficient site was similar to the non-defect group (Fig. 7), thus indicating no inhibition of cartilage repair. On the other hand, when the hydrogel of Comparative Example 2-1 (Fig. 10) or the porous matrix of Comparative Example 2-2 (Fig. 11) was used, the porous matrix remained in the defect lesion and clearly inhibited repair of the subchondral bone. When the porous matrices of Examples 2-3 and 2-4 were used, the results were comparable to using the porous matrix of Example 2-2.

### [Example 3] Preparation of loaded-structures comprising drugs with chondrogenesis-promoting actions supported by biodegradable and/or biocorrosive polymers

### (Example 3-1)

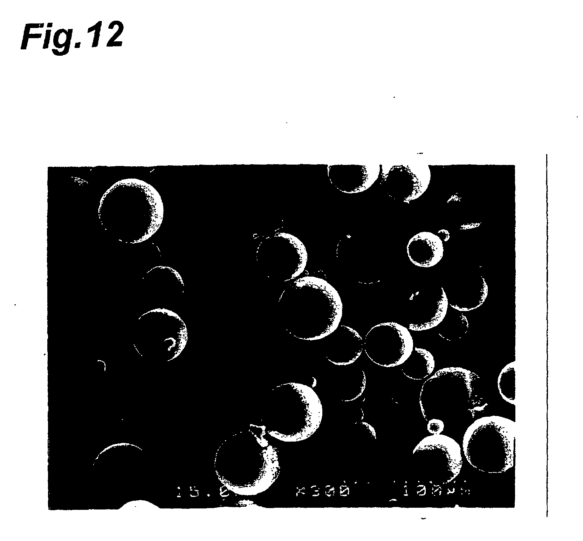

PLGA with a weight-average molecular weight of 10,000 (PLGA7510, Wako Pure Chemical Industries Co., Ltd.) and Compound A were combined in a ratio of PLGA/Compound A = 95/5 (wt/wt), and then 10 mL of methylene chloride (Junsei Chemical Co., Ltd.) was added for dissolution to prepare a 10% (w/v) solution. This was then mixed with 90 mL of 1% aqueous polyvinyl alcohol (87-89% hydrolyzed, MW = 13,000-26,000) (hereinafter, PVA) in a proportion of 1:9 (v/v), and the mixture was stirred with a stirrer at about 700 rpm to yield an O/W emulsion. The emulsion was placed in 900 mL of 1% aqueous PVA, and then the mixture was stirred overnight at about 200 rpm and the solvent was distilled off to yield a suspension of microspheres. The suspension was centrifuged at 1000 rpm, 4°C for 10 minutes, the supernatant was removed, and the precipitate was thoroughly washed with water and freezed to recover the microspheres. Fig. 12 shows a scanning electron micrograph of microspheres comprising Compound A and PLGA7510.

### (Example 3-2)

Microspheres comprising Compound A and PLGA7510 were prepared in the same manner as Example 3-1, except that the proportion of PLGA7510/Compound A was 90/10 (wt/wt).

### (Example 3-3)

Microspheres comprising Compound A and PLGA7510 were prepared in the same manner as Example 3-1, except that the proportion of PLGA7510/Compound A was 80/20 (wt/wt).

### (Example 3-4)

Microspheres comprising Compound A and PLGA7510 were prepared in the same manner as Example 3-1, except that the proportion of PLGA7510/Compound A was 60/40 (wt/wt).

### (Example 3-5) Preparation of PLGA film containing 10% Compound A

PLGA with a weight-average molecular weight of 92,000 (Medisorb7525, Alkermes, Inc.) and Compound A were combined in a ratio of PLGA/Compound A = 90/10 (wt/wt), and then 4 mL of methylene chloride (Junsei Chemical Co., Ltd.) was added for dissolution to prepare a 50% (w/v) solution. After subjecting the solution to ultrasonic irradiation for 5 minutes, it was transferred to a Teflon dish (ϕ50 mm), air dried at room temperature for 3 days and then at 40°C for 7 days, and finally dried *in vacuo* at room temperature for 8 hours, after which a film was recovered.

### (Test Example 3-1)

The microspheres and film of Examples 3-1 to 3-5 were subjected to the following *in vivo* sustained release performance test for Compound A.

### a) Test method

The microspheres were sieved with 38 µm and 75 µm sieves, and the sieved 38-75 µm microspheres were precisely weighed out into approximately 10 mg portions and each placed in a 15 mL Teflon jar. The film was perforated to 5 mm diameters using a cork porer and precisely weighed out to approximately 10 mg portions, and each was placed in a 15 mL Teflon jar. After adding 10 mL of 150 mM PBS (pH 7.4) containing 0.075% Tween80 (polyoxyethylene sorbitan monooleate) and sealing the jar, it was shaken in a 37°C thermostatic bath. This was carried out with n=3 for each of the microspheres and film. At 1 hour, 5 hours and 1, 2, 3, 7, 10, 15, 20, 30, 45 and 60 days after the start of shaking for the microspheres and at 2, 7, 14, 21, 28, 43 and 55 days for the film, the shaking was interrupted, the sample solution was centrifuged and the supernatant solution was used to quantitate Compound A. The solid portion in the centrifugation tube was returned to the Teflon jar, and an additional 10 mL of 150 mM PBS (pH 7.4) containing 0.075% Tween80 was added and shaking was resumed. The concentration of Compound A in the separated supernatant at each time point was quantitated by RP-HPLC to determine the release profile for Compound A from the formulation.

Separately, approximately 10 mg of the microspheres or film was dissolved in 1 mL of acetonitrile in the same manner and diluted 50-fold with acetonitrile. This was also quantitated by RP-HPLC (Waters) and the content of Compound A in each of the microspheres and film was calculated.

### b) HPLC conditions

Column: YMC-Pack ODS-A A-312 150 x 6.0 mm
Column temperature: Room temperature
Sample temperature: 4°C
Detection wavelength: 245 nm
Mobile phase: 60% aqueous acetonitrile
Flow rate: 1.0 mL/min
Injection volume: 20 µL

The quantitation was carried out by the calibration curve method.

### c) Results

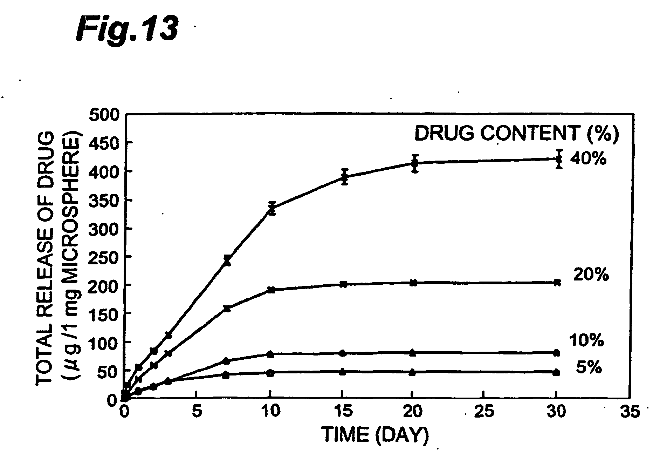

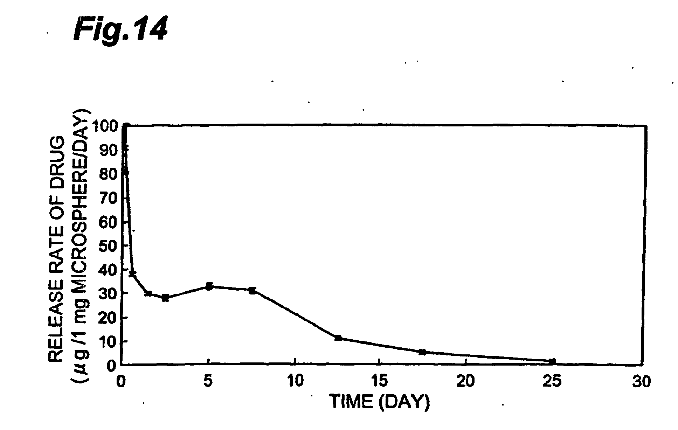

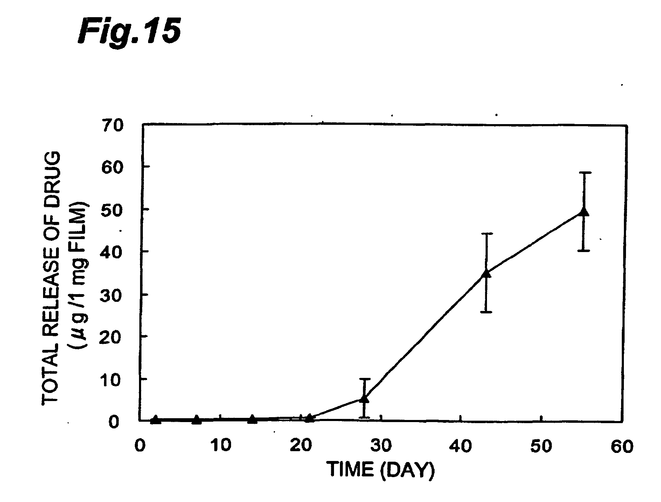

Fig. 13 shows the changes in release of Compound A with time for the microspheres, and Fig. 14 shows the release rate. Fig. 15 shows the change in release of Compound A with time for the film. These graphs indicate that a maximum release rate of about 400 µg/mg can be achieved with microspheres comprising 40% Compound A. The release rate was found to be approximately 5 µg/mg/day over a period of 3 weeks with the microspheres comprising 40% Compound A. With the film comprising 10% Compound A, release began after a period of 20 days. It was also found that the total amounts of Compound A released and the release rates can be controlled by the microsphere particle sizes, the lactic acid/glycolic acid copolymerization ratio and the PLGA molecular weight.

By thus combining a drug having a chondrogenesis-promoting action, a biodegradable and/or biocorrosive polymer and a porous matrix which substantially does not inhibit cartilage tissue repair and/or a hydrogel which substantially does not inhibit cartilage repair (preferably a loaded-structure with the drug supported by the polymer combined with the aforementioned porous matrix and/or hydrogel), it is possible to provide a formulation for cartilage tissue formation which can exhibit a stable and satisfactory cartilage repair effect.

### [Example 4] Preparation of formulation of the invention for cartilage regeneration action experiment in rabbit articular cartilage defect models

### (Example 4-1) Preparation of PLGA films with and without Compound A

PLGA with a weight-average molecular weight of 92,000 (Medisorb7525, Alkermes, Inc.) and Compound A were combined in ratios of PLGA/Compound A = 90/10, 99/1 and 100/0 (wt/wt), and then 4 mL of methylene chloride (Junsei Chemical Co., Ltd.) was added for dissolution to prepare 50% (w/v) solutions. After sonication of each solution for 5 minutes, it was transferred to a Teflon dish (ϕ50 mm), air dried at room temperature for 3 days and then at 40°C for 4 days, and finally dried *in vacuo* at room temperature for 8 hours. The film was perforated to 5 mm diameters using a cork porer and supplied for testing.

### (Example 4-2) Preparation of PLGA microspheres with and without Compound A

PLGA with a weight-average molecular weight of 63,000 (Medisorb5050, Alkermes, Inc.) and Compound A were combined in ratios of PLGA/Compound A = 60/40, 96/4 and 100/0 (wt/wt), and then 40 mL of methylene chloride (Junsei Chemical Co., Ltd.) was added for dissolution to prepare 10% (w/v) solutions. A 10 mL portion of each solution was mixed with 90 mL of 1% aqueous PVA in a proportion of 1:9 (v/v) and stirred with a stirrer at about 700 rpm to yield an O/W emulsion. The emulsion was placed in 900 mL of 1% aqueous PVA, and then the mixture was stirred overnight at about 200 rpm and the solvent was distilled off to yield a suspension of microspheres. The suspension was centrifuged at 1000 rpm, 4°C for 10 minutes, the supernatant was removed, and the precipitate was thoroughly washed with water and freezed to recover the microspheres. These were sieved with 75 µm and 150 µm sieves to yield 75-150 µm microspheres.

### (Example 4-3) Preparation of physically crosslinked hyaluronic acid hydrogel (hydrogen bond-type)

Hyaluronic acid with a weight-average molecular weight of 1.8 x 10⁶ (Suvenyl™, Chugai Pharmaceutical Co., Ltd., dialyzed against distilled water and freezed) was dissolved in distilled water and hydrochloric acid was added to prepare a 1% (w/v) aqueous hyaluronic acid solution with a pH of 1.0. The solution was drawn into a 1 mL syringe with a diameter of about 4 mm at 70 µL portions and frozen at -20°C for 1 week. The samples were then stored at -80°C up until their use for the test.

### (Example 4-4) Preparation of physically crosslinked hyaluronic acid hydrogel encapsulating microspheres prepared in Example 4-2

Approximately 3 mg of the microspheres prepared in Example 4-2 were mixed with 70 µL portions of the 1% (w/v) aqueous hyaluronic acid solutions at pH 1.0 prepared in the same manner as Example 4-3, and the mixtures were stored for use in the test.

### (Text Example 4-1) Evaluation of Compound A contents of film prepared in Example 4-1 and microsphere-encapsulating physically crosslinked hyaluronic acid hydrogel prepared in Example 4-4

### a) Test method

One film prepared in Example 4-1 and one microsphere-encapsulating physically crosslinked hyaluronic acid hydrogel prepared in Example 4-4 were each dissolved in 2 mL of acetonitrile. A 10-fold diluted solution of each in acetonitrile was quantitated by RP-HPLC and the contents of Compound A in the film and microspheres were calculated.

### b) HPLC conditions

The conditions were the same as for Test Example 3-1.

### c) Results

The amounts of Compound A in the microsphere-encapsulating physically crosslinked hyaluronic acid hydrogel are shown in Table 10, and the amounts of Compound A in the film are shown in Table 11.

**(Table 10)**

| Microsphere | | Compound A content (mg/hydrogel) |
|---|---|---|
| Compound A | 0% | 0 |
| Compound A | 4% | 0.10 ±0.01 |
| Compound A | 40% | 0.084 ±0.14 |

**(Table 11)**

| Film | | Compound A content (mg/film) |
|---|---|---|
| Compound A | 0% | 0 |
| Compound A | 1% | 0.11 ±0.003 |
| Compound A | 10% | 1.13 ±0.01 |

### (Test Example 4-2) Drug release test in vitro for film prepared in Example 4-1 and microsphere-encapsulating physically crosslinked hyaluronic acid hydrogel of

### Example 4-4

### a) Test method

One film prepared in Example 4-1 and one microsphere-encapsulating physically crosslinked hyaluronic acid hydrogel prepared in Example 4-4 were each placed in a 15 mL Teflon jar. After adding 10 mL of 150 mM PBS (pH 7.4) containing 0.075% Tween80 and sealing the jar, it was shaken in a 37°C thermostatic bath. At 1, 4, 14, 21, 28, 36 and 50 days after the start of shaking, the shaking was interrupted, the sample solution was centrifuged and the supernatant solution was used to quantitate Compound A. The solid portion in the centrifugation tube was returned to the Teflon jar, and an additional 10 mL of 150 mM PBS (pH 7.4) containing 0.075% Tween80 was added and shaking was resumed. The concentration of Compound A in the separated supernatant at each time point was quantitated by RP-HPLC to determine the release profile for Compound A from the formulation.

### b) HPLC conditions

The conditions were the same as for Test Example 3-1.

### c) Results

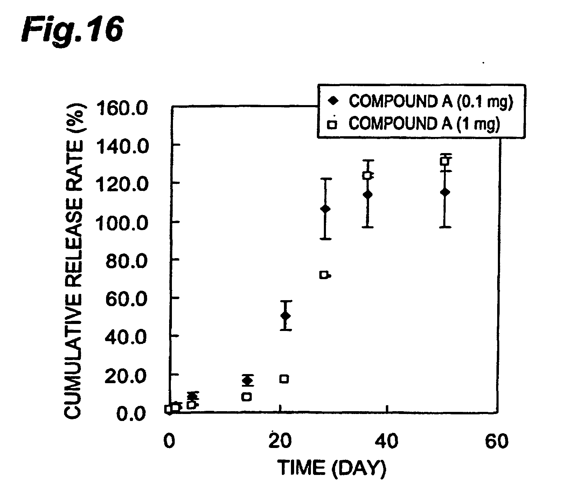

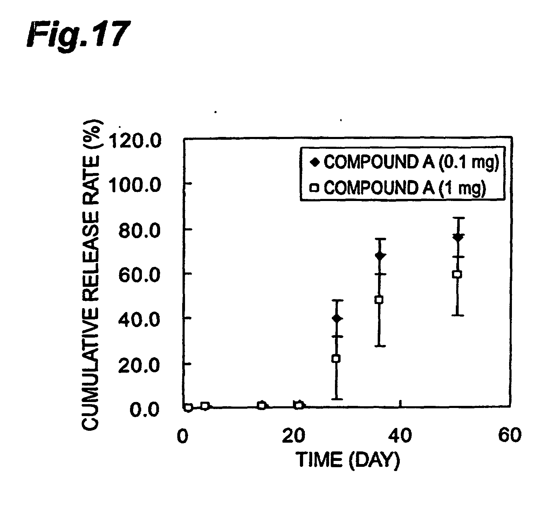

Fig. 16 shows the time-dependent change in the cumulative release rate of Compound A from the microspheres, and Fig. 17 shows the time-dependent change in the cumulative release rate of Compound A from the film.

### [Example 5] Experiment for cartilage regenerating action of invention formulation in rabbit articular cartilage defect models

### (Test Example 5-1) Experiment for cartilage regenerating action of invention formulation in rabbit articular cartilage defect models

### a) Test method

Rabbit articular cartilage defect models were used to examine the cartilage regenerating action of a formulation employing Compound A (WO00/44722) as the chondrogenesis-promoting substance, the physically crosslinked hyaluronic acid of Example 4-3 as the scaffold, and the PLGA film of Example 4-1 or the microspheres of Example 4-2 (a physically crosslinked hyaluronic acid hydrogel encapsulating the microspheres of Example 4-4) for control of release of Compound A.

Using 12-week-old male Kbl:JW rabbits under pentobarbital (Dynabot) anesthesia (40 mg/kg, i.v.), an incision was made in the right articular capsule to expose the femur patellar surface, a hand drill and 5 mm-diameter metal drill blade (Kobelco) were used to create a defect lesion reaching to a 4 mm depth in the bone marrow, the formulation of the invention was implanted, and the articular capsule and skin were sutured. The formulations administered were of the following two types.

Type 1: A formulation comprising the physically crosslinked hyaluronic acid of Example 4-3 filled into the articular cartilage defect lesion of the rabbit, with the PLGA film containing 1 mg of Compound A of Example 4-1 placed thereover.

Type 2: A formulation comprising a physically crosslinked hyaluronic acid hydrogel encapsulating the microspheres of Example 4-4 (physically crosslinked hyaluronic acid hydrogel encapsulating the PLGA microspheres containing 1 mg Compound A prepared in Example 4-2), filled into the articular cartilage defect lesion of the rabbit.

An individual having only the defect was used as a diseased control, and individuals administered a type 1 formulation containing no Compound A (PLGA/compound = 100/0 (wt/wt)) and a type 2 formulation containing no Compound A (PLGA/compound = 100/0 (wt/wt)) were used as vehicle controls. At 3 months after administration of the formulation, the center of each defect lesion was transected with a band saw (Exakt) and demineralized for one week in 5% formic acid (Wako Pure Chemical Industries Co., Ltd.). This was followed by neutralization overnight with a 20% sodium acetate solution and preparation of paraffin sections according to an ordinary method. The sections were stained with Safranin O-Fast Green and observed with an optical microscope (Nikon) for histological evaluation of subchondral bone repair in the defect lesions.

### b) Results

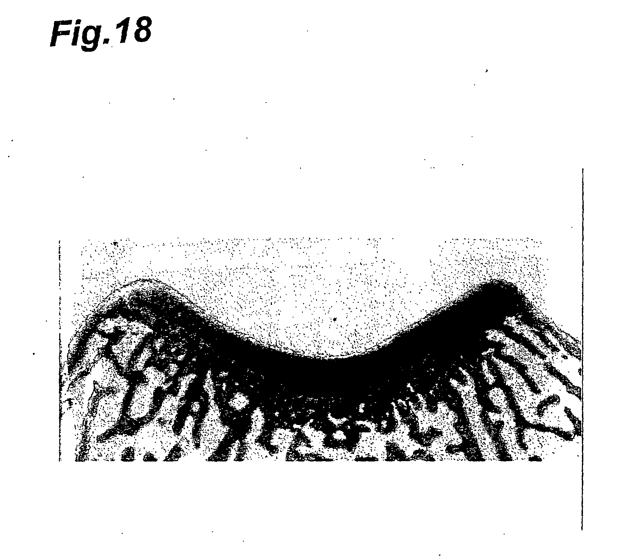

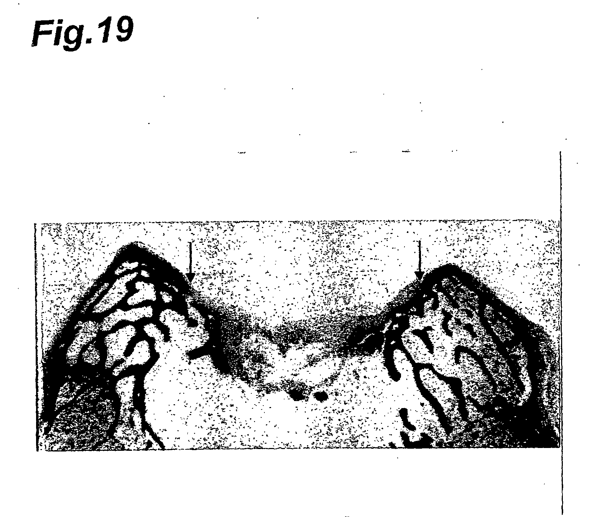

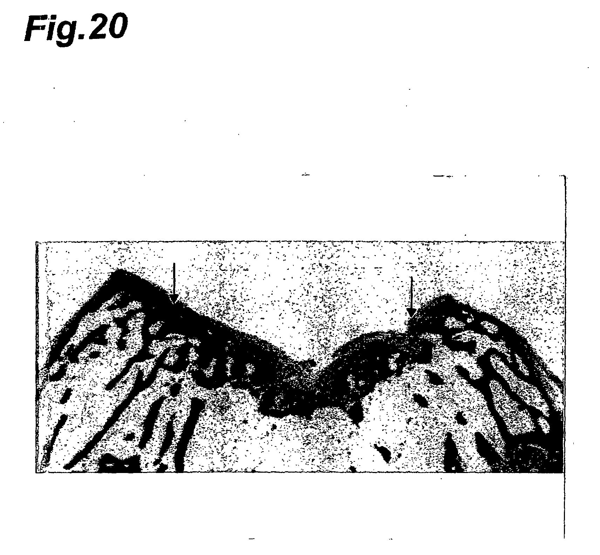

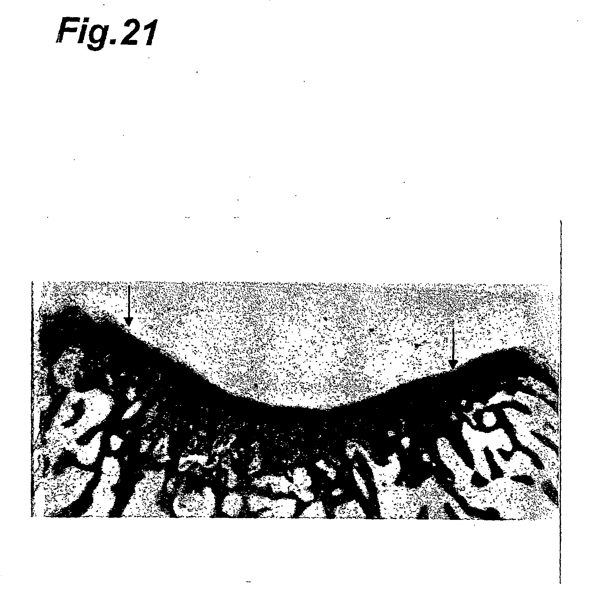

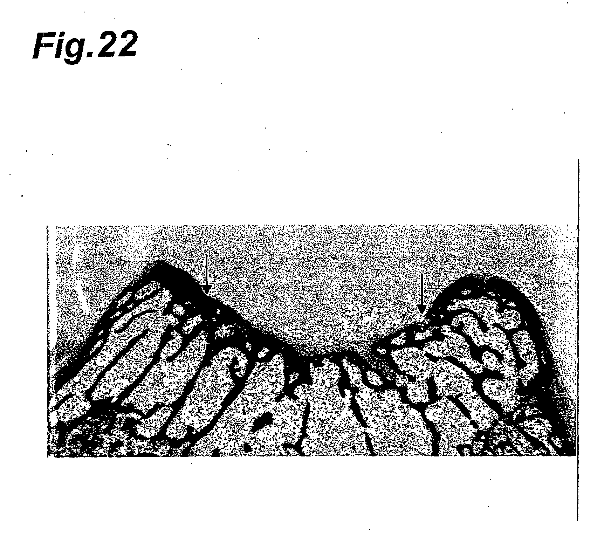

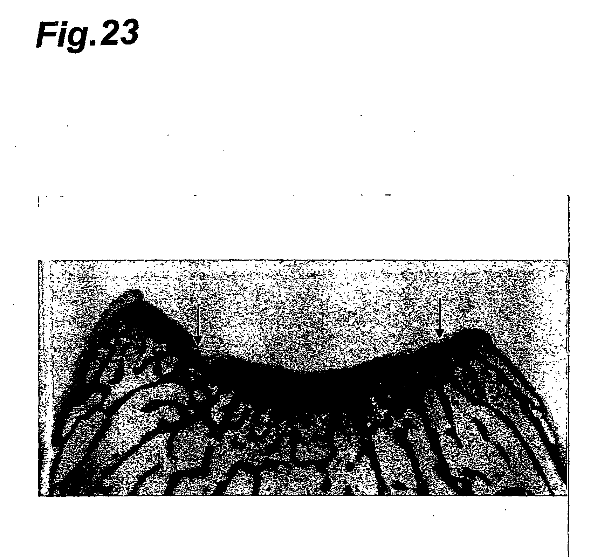

The results for the tissue samples are shown in Figs. 18 to 23. Fig. 18 shows normal tissue with no articular cartilage defect, and Fig. 19 shows tissue from an individual having only the defect. Fig. 20 shows tissue from an individual administered the type 1 formulation containing no Compound A (vehicle control), Fig. 21 shows the same for the type 1 formulation containing 1 mg of Compound A, Fig. 22 shows the same for the type 2 formulation containing no Compound A (solvent control), and Fig. 23 shows the same for the type 2 formulation containing 1 mg of Compound A. In the photographs, the areas between the arrows indicate the defect lesions (with the arrows indicating the borders between the defect lesions and normal sites).

As clearly seen in Fig. 18, absolutely no hyaline cartilage was regenerated at the defect lesion in the individual having only the defect. The type 1 (Fig. 20) and type 2 (Fig. 22) formulations (solvent controls) which contained no Compound A induced no hyaline cartilage regeneration for the defect. In contrast, both of the formulations containing 1 mg of Compound A (type 1 (Fig. 21) and type 2 (Fig. 23)) induced regeneration of hyaline cartilage at the defect lesions. These results demonstrated that the formulation of the invention causes regeneration of original cartilage tissue at cartilage defect lesions which do not undergo natural repair.

### Industrial Applicability

As explained above, the formulation for cartilage tissue formation of the present invention comprises a drug having a chondrogenesis-promoting action, a biodegradable and/or biocorrosive polymer and a porous matrix which substantially does not inhibit cartilage tissue repair and/or a hydrogel which substantially does not inhibit cartilage repair to serve as scaffolding for cartilage tissue repair, and therefore the formulation has excellent biocompatibility, while also being stable and capable of producing a satisfactory cartilage repair effect without requiring highly skilled techniques or extensive equipment for treatment. Because this has not been achievable with conventional formulations, the formulation of the invention has very high practical utility.

## Claims

1. A formulation for cartilage tissue formation, comprising a drug having a chondrogenesis-promoting action, a biodegradable and/or biocorrosive polymer, and a porous matrix and/or a hydrogel, wherein the porus matrix and the hydrogel substantially do not inhibit cartilage repair.

2. A formulation for cartilage tissue formation according to claim 1, wherein said polymer is a polymer which controls release of said drug *in vivo,* and said porous matrix and said hydrogel are scaffold-forming materials which serve as scaffolding for cartilage tissue formation *in vivo.*

3. A formulation for cartilage tissue formation according to claim 2, wherein said drug and said polymer form a loaded-structure with said drug supported by said polymer, and said scaffold-forming material is in contact with said loaded-structure.

4. A formulation for cartilage tissue formation according to claim 3, wherein said loaded-structure is dispersed in said scaffold-forming material.

5. A formulation for cartilage tissue formation according to claim 3, wherein said scaffold-forming material forms a layer, and said loaded-structure is fixed on said layer.

6. A formulation for cartilage tissue formation according to any one of claims 3 to 5, wherein said loaded-structure is in the form of a microsphere, a film or nonwoven fabric.

7. A formulation for cartilage tissue formation according to claim 6, wherein said microsphere is obtained from an aqueous dispersion of said drug and said polymer in an organic solvent solution, by removing the organic solvent and water.

8. A formulation for cartilage tissue formation according to any one of claims 3 to 5, wherein said loaded-structure is a loaded-structure capable of sustained release of said drug.

9. A formulation for cartilage tissue formation according to claim 4, wherein said scaffold-forming material is said hydrogel.

10. A formulation for cartilage tissue formation according to claim 5, wherein said scaffold-forming material is said hydrogel, and said loaded-structure is in the form of a film or nonwoven fabric.

11. A formulation for cartilage tissue formation according to claim 1, wherein said drug is the compound represented by the general formula (I) or a salt thereof: 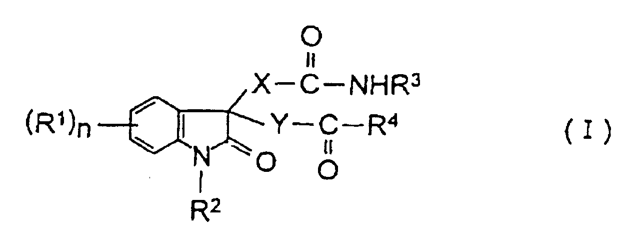 wherein
R¹ represents a halogen atom, a lower alkyl group, a lower alkoxy group, a hydroxyl group, a nitro group, a trifluoromethyl group, a lower alkylthio group, an acyl group, a carboxyl group, a mercapto group or an amino group with an optional substituent;
R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent or a heterocyclic group with an optional substituent;
R³ represents a lower alkyl group with an optional substituent, a cycloalkyl group with an optional substituent, an aryl group with an optional substituent or a heterocyclic group with an optional substituent;
R⁴ represents a hydrogen atom, a lower alkyl group with an optional substituent, an aryl group with an optional substituent, a heterocyclic group with an optional substituent, -OR⁵, -SR⁵ or -NR⁶R⁷ wherein R⁵, R⁶ and R⁷ may be the same or different and each represents a hydrogen atom, a lower alkyl group with an optional substituent, a cycloalkyl group with an optional substituent, an aryl group with an optional substituent, a heterocyclic group with an optional substituent, a lower alkoxy group or an amino group with an optional substituent, and R⁶ and R⁷ may together form a group represented by -(CH₂)ₘ- or -(CH₂)ₗNR⁸(CH₂)ₖ- wherein k, l and m each represent an integer of 1-8 and R⁸ represents a hydrogen atom or a lower alkyl group; and
X and Y may be the same or different and each represents -CH₂-, -NH- or -O-, and n represents an integer of 0-4.

12. A formulation for cartilage tissue formation according to claim 11, wherein the compound represented by the general formula (I) is the compound represented by the following formula (A).

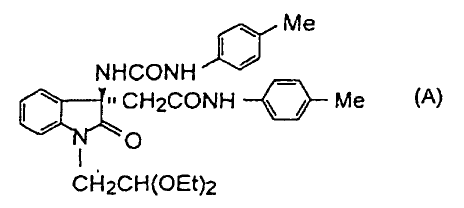

13. A formulation for cartilage tissue formation according to claim 1, wherein said polymer has a weight-average molecular weight of 500 or greater.

14. A formulation for cartilage tissue formation according to claim 1, wherein said polymer is one or more polymers selected from the group consisting of a polyhydroxyalkanoic acid, a lactone ring-opened polymer, a hydroxyalkanoic acid/lactone copolymer, a polyacetal, a polyketal, a polymethylvinyl ether, chitin, chitosan, a polyamide, a polyamino acid, a polyurethane, a polyester amide, a polycarboxylic acid, a polyanhydride, a polyalkylene, a polyphosphoric acid, a polyorthoester, and a copolymer comprising one or more of the foregoing in the molecule.

15. A formulation for cartilage tissue formation according to claim 1, wherein said polymer is a polymer or copolymer of a compound selected from the group consisting of glycolic acid, lactic acid, hydroxybutyric acid and hydroxyvaleric acid.

16. A formulation for cartilage tissue formation according to claim 1, wherein said polymer is a lactic acid/glycolic acid copolymer.

17. A formulation for cartilage tissue formation according to claim 1, wherein at least a part of said polymer is a reactive polymer having an unsaturated double bond.

18. A formulation for cartilage tissue formation according to claim 17, wherein said reactive polymer is a compound comprising a backbone and at least one (meth)acryloyl group bonded to the backbone, wherein the backbone comprises one or more polymers selected from the group consisting of a polyhydroxyalkanoic acid, a lactone ring-opened polymer, a hydroxyalkanoic acid/lactone copolymer, a polyacetal, a polyketal, a polymethylvinyl ether, chitin, chitosan, a polyamide, a polyamino acid, a polyurethane, a polyester amide, a polycarboxylic acid, a polyanhydride, a polyalkylene, a polyphosphoric acid, a polyorthoester, and a copolymer comprising one or more of the foregoing in the molecule.

19. A formulation for cartilage tissue formation according to claim 1, wherein said porous matrix is a porous matrix having a mean pore size of 10-500 µm.

20. A formulation for cartilage tissue formation according to claim 1, wherein said porous matrix is a porous matrix having an *in vivo* elimination rate of 1 day to 5 weeks.

21. A formulation for cartilage tissue formation according to claim 1, wherein said porous matrix is a porous matrix comprising a biodegradable and/or biocorrosive compound.

22. A formulation for cartilage tissue formation according to claim 1, wherein said biodegradable and/or biocorrosive compound has a weight-average molecular weight of 500 or greater.

23. A formulation for cartilage tissue formation according to claim 21, wherein said biodegradable and/or biocorrosive compound is a hyaluronic acid derivative and/or collagen.

24. A formulation for cartilage tissue formation according to claim 23, wherein said hyaluronic acid derivative is physically crosslinked hyaluronic acid or chemically crosslinked hyaluronic acid with low-crosslinked density.

25. A formulation for cartilage tissue formation according to claim 24, wherein said physically crosslinked hyaluronic acid is hydrogen bond-type physically crosslinked hyaluronic acid or ionic bond-type physically crosslinked hyaluronic acid.

26. A formulation for cartilage tissue formation according to claim 21, wherein said biodegradable and/or biocorrosive compound is one or more polymers selected from the group consisting of a polyhydroxyalkanoic acid, a lactone ring-opened polymer, a hydroxyalkanoic acid/lactone copolymer, a polyacetal, a polyketal, a polymethylvinyl ether, chitin, chitosan, a polyamide, a polyamino acid, a polyurethane, a polyester amide, a polycarboxylic acid, a polyanhydride, a polyalkylene, a polyphosphoric acid, a polyorthoester, and a copolymer comprising one or more of the foregoing in the molecule.

27. A formulation for cartilage tissue formation according to claim 21, wherein said biodegradable and/or biocorrosive compound is a polymer or copolymer of a compound selected from the group consisting of glycolic acid, lactic acid, hydroxybutyric acid and hydroxyvaleric acid.

28. A formulation for cartilage tissue formation according to claim 21, wherein said biodegradable and/or biocorrosive compound is a lactic acid/glycolic acid copolymer.

29. A formulation for cartilage tissue formation according to claim 21, wherein at least a part of said biodegradable and/or biocorrosive compound is a reactive polymer having an unsaturated double bond.

30. A formulation for cartilage tissue formation according to claim 29, wherein said reactive polymer is a compound comprising a backbone and at least one (meth)acryloyl group bonded to the backbone, wherein the backbone comprises one or more polymers selected from the group consisting of a polyhydroxyalkanoic acid, a lactone ring-opened polymer, a hydroxyalkanoic acid/lactone copolymer, a polyacetal, a polyketal, a polymethylvinyl ether, chitin, chitosan, a polyamide, a polyamino acid, a polyurethane, a polyester amide, a polycarboxylic acid, a polyanhydride, a polyalkylene, a polyphosphoric acid, a polyorthoester, and a copolymer comprising one or more of the foregoing in the molecule.

31. A formulation for cartilage tissue formation according to claim 1, wherein said hydrogel is a hydrogel having an *in vivo* elimination rate of 1 day to 5 weeks.

32. A formulation for cartilage tissue formation according to claim 31, wherein said hydrogel is a hydrogel capable of gelling *in situ*.

33. A method of forming cartilage tissue comprising introducing into a cartilage defect lesion a formulation for cartilage tissue formation according to claim 4.

34. A method of forming cartilage tissue comprising introducing into a cartilage defect lesion a formulation for cartilage tissue formation according to claim 5 in such a manner that said layer is in contact with the surface of said cartilage defect lesion.
